# EUROPEAN PATENT SPECIFICATION

(11) **EP 3 579 836 B1**
(45) Date of publication and mention of the grant of the patent: **15.12.2021**
(21) Application number: 18750617.5
(22) Date of filing: 09.02.2018
(51) Int. Cl.: A61K 31/501, A61K 45/06, A61P 25/00

(54) **METHODS FOR AUTISM SPECTRUM DISORDER PHARMACOTHERAPY**
VERFAHREN ZUR MEDIKAMENTÖSEN THERAPIE VON STÖRUNGEN DES AUTISMUSSPEKTRUMS
PROCÉDÉS POUR LA PHARMACOTHÉRAPIE DE TROUBLES DU SPECTRE AUTISTIQUE

(30) Priority: 09.02.2017 US 201762457120 P
(43) Date of publication of application: 18.12.2019
(73) Proprietor: Perfect Daylight Limited, MC 98000 Monaco (MC); The Regents of the University of California, Oakland, CA 94607-5200 (US)
(72) Inventor: VAUGHAN, Brent, Portola Valley California 94028 (US); NAVIAUX, Robert K., San Diego California 92124 (US)
(74) Representative: J A Kemp LLP
(86) International application number: PCT/US2018/017674
(87) International publication number: WO 2018/148580

(56) References cited:
- WO-A1-2012/082056
- US-A- 5 728 684
- US-A1- 2016 209 428
- US-A1- 2016 339 065
- US-B2- 8 633 250
- JANE C NAVIAUX ET AL: "Antipurinergic therapy corrects the autism-like features in the Fragile X (Fmr1 knockout) mouse model", MOLECULAR AUTISM, BIOMED CENTRAL LTD, LONDON, UK, vol. 6, no. 1, 13 January 2015 (2015-01-13), page 1, XP021212643, ISSN: 2040-2392, DOI: 10.1186/2040-2392-6-1
- I NAVIAUX et al.: "Reversal of autism-like behaviors and metabolism in adult mice with single-dose antipurinergic therapy", Translational Psychiatry, vol. 4, no. 6, 1 June 2014 (2014-06-01), page e400, XP055534566,
- NAVIAUX et al.: "Antipurinergic Therapy Corrects the Autism-Like Features in the Poly(IC) Mouse Model", Plos One, vol. 8, no. 3, 13 March 2013 (2013-03-13), page e57380, XP055534571,
- HAMIDPOUR et al.: "Antipurinergic Therapy with Suramin as a Treatment for Autism Spectrum Disorder", Journal of Biomedical Sciences, vol. 5, no. 2, 29 March 2016 (2016-03-29), pages 1-7, XP055534575,
- OGDEN et al.: "Suramin as a Chemosensitizer: Oral Pharmacokinetics in Rats", Pharmaceutical Research, vol. 21, no. 11, November 2004 (2004-11), pages 2058-2063, XP055534576,
- ZHUANG et al.: "Suramin promotes recovery from renal ischemia/reperfusion injury in mice", Kidney International, vol. 75, no. 3, February 2009 (2009-02), pages 304-311, XP055534580,
- EICHHORST et al.: "Suramin inhibits death receptor-induced apoptosis in vitro and fulminant apoptotic liver damage in mice", Nature Medicin e, vol. 10, no. 6, 16 May 2004 (2004-05-16), pages 602-609, XP055534584,

## Description

### BACKGROUND

Although the CDC estimates that 1 in 68 children in the United States have an autism spectrum disorder (ASD), molecular understanding and treatment of the disease has been lacking. No single gene or copy number variation is associated with ASD in 100% of children who carry the mutation, and no single DNA mutation account for more than 1-2% of all ASD. While specific environmental factors have also been shown to increase the risk of ASD, no single child with ASD has all of the known genetic risk factors, or is exposed to all the same environmental triggers. In addition, no single chemical, anatomical or physiological biomarker has yet been identified that is present in all persons with ASD.

Naviaux et al. (Molecular Autism 2015, 6:1) describes a study on the effect of suramin on a Fragile X (*Fmr1*) knockout mouse model.

### SUMMARY OF THE INVENTION

Any reference to a method of treatment in this description is to be regarded as referring to an agent for use in a method of treatment.

There is need for identification of general biological processes that underlie the symptoms of ASD as well as classes of therapeutic agents that are effective in treating the disorder in ASD patients regardless of known genetic or environmental risk factors. The present disclosure remedies this deficiency with compounds and compositions for treating ASD and cognitive developmental disorders.

The invention is defined in claim 1. Further aspects and preferred embodiments are defined in the dependent claims. Any aspects, embodiments and examples of the present disclosure which do not fall under the scope of the appended claims do not form part of the invention and are merely provided for illustrative purposes.

In some aspects, the invention provides an antipurinergic agent for use in a method of treating an autism spectrum disorder in a subject in need thereof, wherein the method comprises administering the antipurinergic agent to said subject in an amount sufficient to maintain the antipurinergic agent plasma levels of said subject within a range of from about 3 µM to about 30 µM for 21 days or more. In one embodiment, the antipurinergic agent plasma levels of said subject are maintained within a range of from about 3 µM to about 25 µM for 21 days or more. In some embodiments, the antipurinergic agent plasma levels of said subject are maintained within a range of from about 3 µM to about 15 µM for 21 days or more. In some embodiments, the antipurinergic agent plasma levels are maintained within a range of from about 3 µM to about 15 µM for 28 days or more. In some embodiments, the antipurinergic agent plasma levels are maintained within a range of from about 3 µM to about 15 µM for 45 days or more. In some embodiments, the antipurinergic agent plasma levels are maintained with a range of from about 5 µM to about 15 µM. In some embodiments, the administration occurs two or more times. In some embodiments, the antipurinergic agent is administered intravenously. In some embodiments, the antipurinergic agent is administered orally, subcutaneously, intramuscularly, by inhalation, cutaneously, or transdermally. In some embodiments, the antipurinergic agent is not administered intravenously.

Also disclosed herein is a method of treating an autism spectrum disorder in a subject in need thereof, comprising administering an antipurinergic agent to the subject in an amount sufficient to maintain the antipurinergic agent plasma levels of said subject from about 1 µM to about 30 µM for at least about 21 days, wherein the antipurinergic agent is not administered intravenously. In some aspects of this disclosure, the antipurinergic agent plasma levels of the subject are maintained within a range of from about 1 µM to about 15 µM for 21 days or more. In some aspects, the administration is selected from orally, subcutaneously, intramuscularly, rectally, by inhalation, by nebulization, cutaneously, and transdermally. In some aspects, the administration is subcutaneously. In some embodiments, the administration is orally. In some aspects, the antipurinergic agent plasma levels are maintained from about 5 µM to about 15 µM.

In some aspects, the invention provides an antipurinergic agent for use in a method of treating an autism spectrum disorder in a subject in need thereof, wherein the method comprises administering the antipurinergic agent to the subject in an amount sufficient to maintain the antipurinergic agent plasma levels of the subject from about 1 µM to about 30 µM for at least about 21 days, wherein the antipurinergic agent is administered in two or more doses. In some embodiments, the antipurinergic agent plasma levels of the subject are maintained within a range of from about 1 µM to about 15 µM for 21 days or more. In some embodiments, the antipurinergic agent is administered in three or more doses. In some embodiments, the antipurinergic agent plasma levels are maintained from about 5 µM to about 15 µM. In some embodiments, the doses of antipurinergic agent are administered intravenously. In some embodiments, the doses of antipurinergic agent are administered orally, subcutaneously, intramuscularly, by inhalation, cutaneously, or transdermally. In some embodiments, the doses of antipurinergic agent are not administered intravenously.

Also disclosed herein is a method of treating an autism spectrum disorder in a subject in need thereof, comprising administering an antipurinergic agent to the subject, testing the subject's antipurinergic agent plasma level at one or more time points after said administration, evaluating the antipurinergic agent plasma levels from the one or more time points to determine a treatment regimen for the subject in an amount sufficient to achieve antipurinergic agent plasma levels from about 1 µM to about 30 µM. In some aspects of this disclosure, the treatment regimen for said subject is in an amount sufficient to achieve antipurinergic agent plasma levels from about 1 µM to about 15 µM. In some aspects, the treatment regimen for the subject is in an amount sufficient to achieve antipurinergic agent plasma levels from about 5 µM to about 15 µM. In some aspects, the method further comprises administering to the subject one or more doses of the antipurinergic agent in an amount sufficient to maintain plasma levels from about 1 µM to about 30 µM for 21 days or more. In some aspects, the administering to the subject one or more doses of said antipurinergic agent is in an amount sufficient to maintain plasma levels from about 1 µM to about 30 µM for 40 days or more. In some aspects, the plasma levels are evaluated periodically such as once every 5 days, once a week, once every two weeks, once every three weeks, or once every four weeks.

In some aspects, the invention provides an antipurinergic agent for use in a method of treating an autism spectrum disorder in a subject in need thereof, wherein the method comprises administering the antipurinergic agent to the subject in a first dose followed by a 10-40 day treatment holiday and then administering a second dose of the antipurinergic agent. In some embodiments, the first dose is within a range from about 10 mg/kg to about 30 mg/kg. In some embodiments, the second dose is within a range from about 10 mg/kg to about 30 mg/kg. In some embodiments, the first dose is within a range from about 400 mg/m² to about 600 mg/m². In some embodiments, the second dose is within a range from about 400 mg/m² to about 600 mg/m². In some embodiments, the treatment holiday is within a range from about 12 to about 20 days. In some embodiments, the method further comprises a 10-60 day treatment holiday after the second dose, followed by a third dose. In some embodiments, the second dose and the third dose are the same. In some embodiments, the third dose is smaller than the second dose. In some embodiments, the third dose is smaller than the second dose, and within a range from about 10 mg/kg to about 30 mg/kg. In some embodiments, the third dose is smaller than the second dose, and within a range from about 400 mg/m² to about 600 mg/m².

Also disclosed herein is a method of treating an autism spectrum disorder in a subject in need thereof, wherein the treatment comprises administering to said subject a dose of an antipurinergic agent of more than 20 mg/kg. In some aspects of this disclosure, the subject is administered a dose of an antipurinergic agent of from about 21 mg/kg to about 40 mg/kg. In some aspects, the antipurinergic agent is administered in an amount sufficient to maintain serum creatinine levels of the subject below 1.3 mg/dL following the administration. In some aspects, the antipurinergic agent is administered in an amount sufficient to maintain serum creatinine levels of the subject below 1.0 mg/dL following the administration. In some aspects, the antipurinergic agent is administered in an amount sufficient to maintain serum aminotransferase levels of the subject below 40 U/L following the administration.

Also disclosed herein is a method of treating an autism spectrum disorder in a subject in need thereof, wherein the treatment comprises administering to the subject a dose of an antipurinergic agent of more than 300 mg/m². In some aspects of this disclosure, the subject is administered a dose of an antipurinergic agent of from about 350 mg/m² to about 600 mg/m². In some aspects, the antipurinergic agent is administered in an amount sufficient to maintain serum creatinine levels of the subject below 1.3 mg/dL following the administration. In some aspects, the antipurinergic agent is administered in an amount sufficient to maintain serum creatinine levels of the subject below 1.0 mg/dL following the administration. In some aspects, the antipurinergic agent is administered in an amount sufficient to maintain serum aminotransferase levels of the subject below 40 U/L following the administration.

Also disclosed herein is a method of treating an autism spectrum disorder in a subject in need thereof, comprising administering an antipurinergic agent to the subject in a first dose of less than 20 mg/kg, followed by a 2-20 day treatment holiday and then administering a second dose of the antipurinergic agent. In some aspects of this disclosure, the first dose of antipurinergic agent is selected from about 1 mg/kg to about 19 mg/kg. In some aspects, the second dose of antipurinergic agent is selected from about 1 mg/kg to about 19 mg/kg. In some aspects, the antipurinergic agent is administered in an amount sufficient to maintain the antipurinergic agent plasma levels of the subject within a range of from about 1 µM to about 15 µM for 21 days to about 50 days.

Also disclosed herein is a method of treating an autism spectrum disorder in a subject in need thereof, comprising administering an antipurinergic agent to the subject in a first dose of less than 350 mg/m², followed by a 2-20 day treatment holiday and then administering a second dose of the antipurinergic agent. In some aspects of this disclosure, the first dose of antipurinergic agent is selected from about 50 mg/m² to about 300 mg/m². In some aspects, the second dose of antipurinergic agent is selected from about 50 mg/m² to about 600 mg/m². In some aspects, the antipurinergic agent is administered in an amount sufficient to maintain the antipurinergic agent plasma levels of the subject within a range of from about 1 µM to about 25 µM for 21 days to about 50 days.

Also disclosed herein is a method of identifying a subject that would benefit from treatment with an antipurinergic agent, comprising identifying a subject with an autism spectrum disorder, wherein the autism spectrum disorder is not a known syndromic form of autism spectrum disorder caused by DNA mutation or chromosomal copy number variation (CNV). In some aspects of this disclosure, the method further comprises treating said subject by administering an antipurinergic agent in response to the step of identifying.

Also disclosed herein is a method of identifying a subject that would benefit from treatment with an antipurinergic agent, comprising identifying a subject with an autism spectrum disorder, wherein the autism spectrum disorder is a known syndromic form of autism spectrum disorder caused by DNA mutation or chromosomal copy number variation (CNV). In some aspects of this disclosure, the method further comprises treating the subject by administering an antipurinergic agent in response to the step of identifying.

Also disclosed herein is a method of identifying a subject that would benefit from treatment with an antipurinergic agent, comprising identifying a subject with an autism spectrum disorder, wherein the subject has one or more symptoms of a gastrointestinal disease or disorder. In some aspects of this disclosure, the method further comprises treating the subject by administering an antipurinergic agent in response to the step of identifying. In some aspects, the antipurinergic agent comprises suramin, a salt thereof, or a prodrug thereof. In some aspects, the subject is a child. In some aspects, the subject is 18 years old or younger. In some aspects, the method further comprises a step of identifying a subject with an autism spectrum disorder, wherein the autism spectrum disorder is a known syndromic form of autism spectrum disorder caused by DNA mutation or chromosomal copy number variation (CNV), wherein the step is performed prior to the administering. In some aspects, the administering is in response to the step of identifying. In some aspects, the method further comprises a step of identifying a subject with an autism spectrum disorder, wherein the autism spectrum disorder is not a known syndromic form of autism spectrum disorder caused by DNA mutation or chromosomal copy number variation (CNV), wherein the step is performed prior to the administering. In some aspects, the subject is treated in response to the step of identifying. In some aspects, the autism spectrum disorder includes one or more symptoms selected from difficulty communicating, difficulty interacting with others, and repetitive behaviors. In some aspects, treating the autism spectrum disorder comprises improving one or more symptoms relative to symptoms of the subject prior to the administration, wherein the symptoms are selected from difficulty communicating, difficulty interacting with others, and repetitive behaviors. In some aspects, treating the autism spectrum disorder comprises improving an assessment score of the subject selected from ADOS, ABC, ATEC, and CGI scores relative to a score from the subject prior to the administration. In some aspects, an assessment score of the subject selected from ADOS, ABC, ATEC, and CGI scores is improved by 10% or more relative to a score from the subject prior to the administration. In some aspects, an ADOS score of the subject is improved by 1.6 or more relative to a score prior to the administration, or a corresponding performance improvement on a similar test. In some aspects, the p-value of the performance improvement on the ADOS or similar test is 0.05 or less. In some aspects, the effect size of the improvement on the ADOS or similar test is about 1 or more. In some aspects, the effect size is about 2.9 or more. In some aspects, following the administration the AUC is from about 80 µM*days to about 400 µM*days.

Also disclosed herein is a pharmaceutical composition comprising a prodrug of suramin and a pharmaceutically acceptable excipient.

Also disclosed herein is a pharmaceutical composition suitable for intravenous administration, wherein the composition comprises suramin or a salt thereof and one or more excipients selected from antimicrobials, polyethylene glycol, stabilizers, and antioxidants.

Also disclosed herein is a pharmaceutical composition, comprising suramin, a delivery system that increases oral absorption of suramin and a pharmaceutically acceptable excipient. In some aspects of this disclosure, the delivery system comprises a polymeric delivery system. In some aspects, the polymeric delivery system comprises liposomes, nanoparticles, or microspheres.

### BRIEF DESCRIPTION OF THE DRAWINGS

The features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

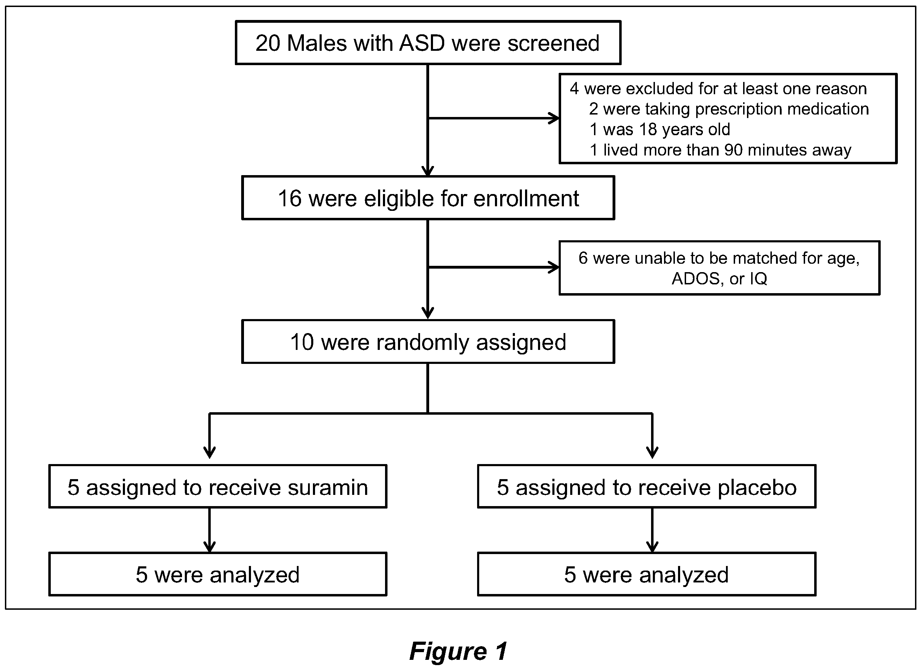

**Fig. 1** shows the patient selection design for the SAT-1 trial of suramin in autism spectrum disorder.

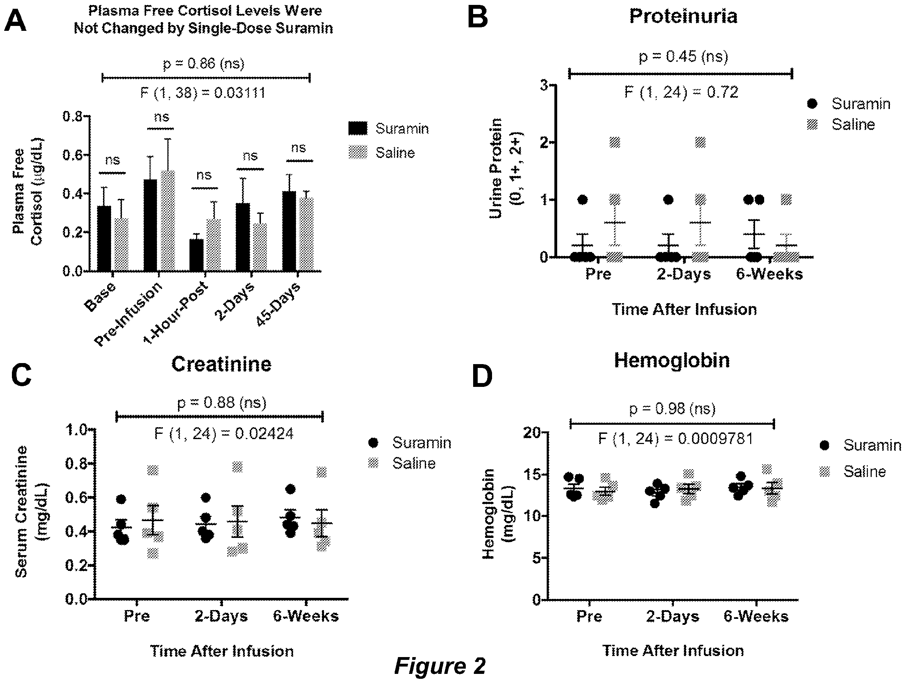

**Fig. 2** depicts monitoring of safety parameters for suramin dosing for patients of the SAT-1 trial. (A) shows free cortisol, (B) shows proteinuria, (C) shows creatinine, and (D) shows hemoglobin. Data were analyzed by 2-way ANOVA to test for treatment, time, and treatment x time interaction effects. P and F values reflect the treatment effect. No significant effects were found on these measures.

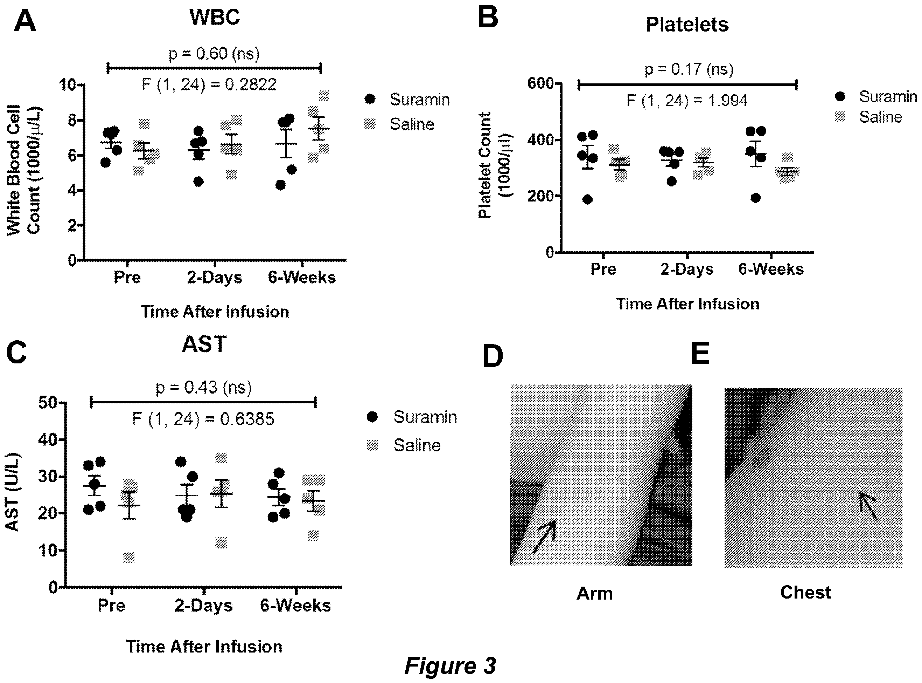

**Fig. 3** depicts further monitoring of safety parameters for suramin dosing for patients of the SAT-1 trial. (A) shows white blood cells (WBC), (B) shows platelets, (C) shows aspartate aminotransferase (AST), (D) shows rash-antecubital fossa morphology, and (I) shows rashchest morphology. Data were analyzed by 2-way ANOVA to test for treatment, time, and treatment x time interaction effects. P and F values reflect the treatment effect. Only the rash was significantly different between suramin and placebo groups.

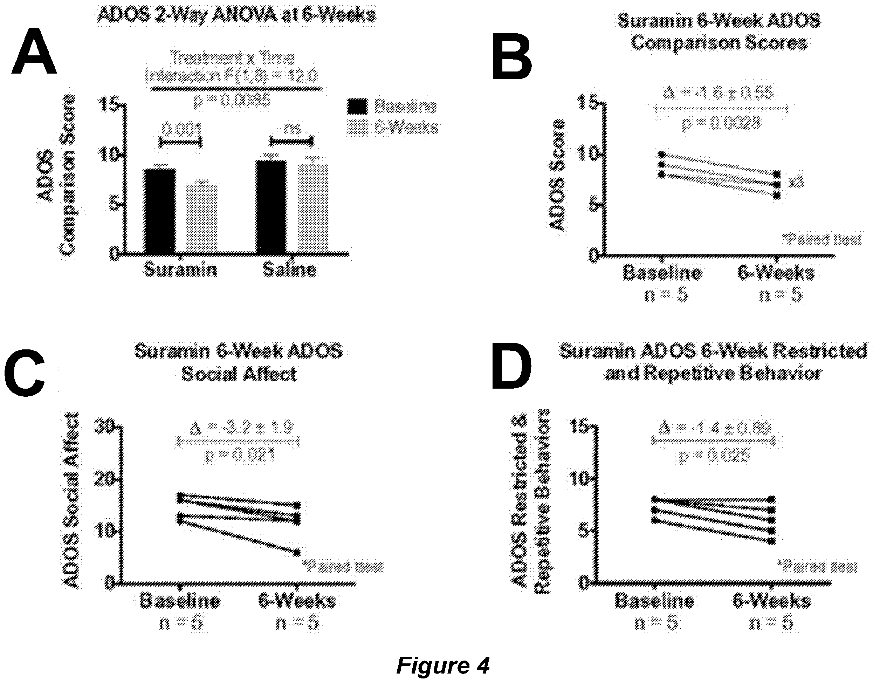

**Fig. 4** depicts overall ADOS score effects at 6-weeks and suramin effects on ADOS submeasures at 6-weeks. (A) shows 6-week ADOS comparison scores for suramin and saline by 2-Way ANOVA, (B) shows 6-week ADOS comparison score improvement after Suramin, (C) shows 6-Week ADOS Social Affect score improvement after Suramin, and (D) shows 6-Week ADOS Restricted and Repetitive Behavior score improvement after Suramin.

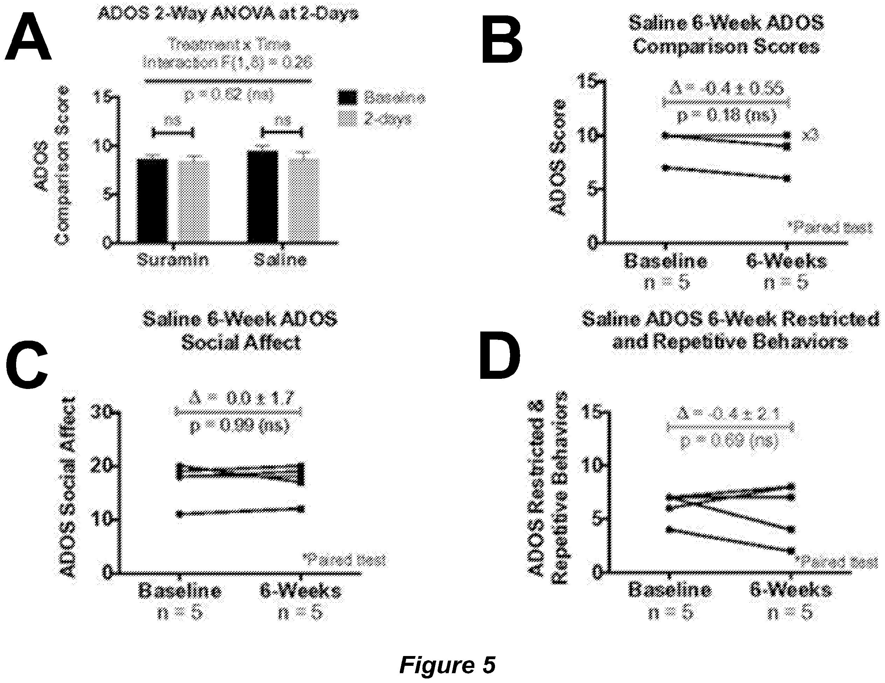

**Fig. 5** depicts overall ADOS score effects at 2-days and saline (placebo) effects on ADOS submeasures at 6-weeks. (A) shows that 2-Day ADOS comparison scores were not changed, (B) shows no change in 6-Week ADOS Scores in subjects receiving saline placebo, (C) shows no change in 6-Week ADOS Social Affect Scores in subjects receiving placebo, and (D) shows no change in 6-Week ADOS Restricted and Repetitive Behavior Scores in subjects receiving placebo.

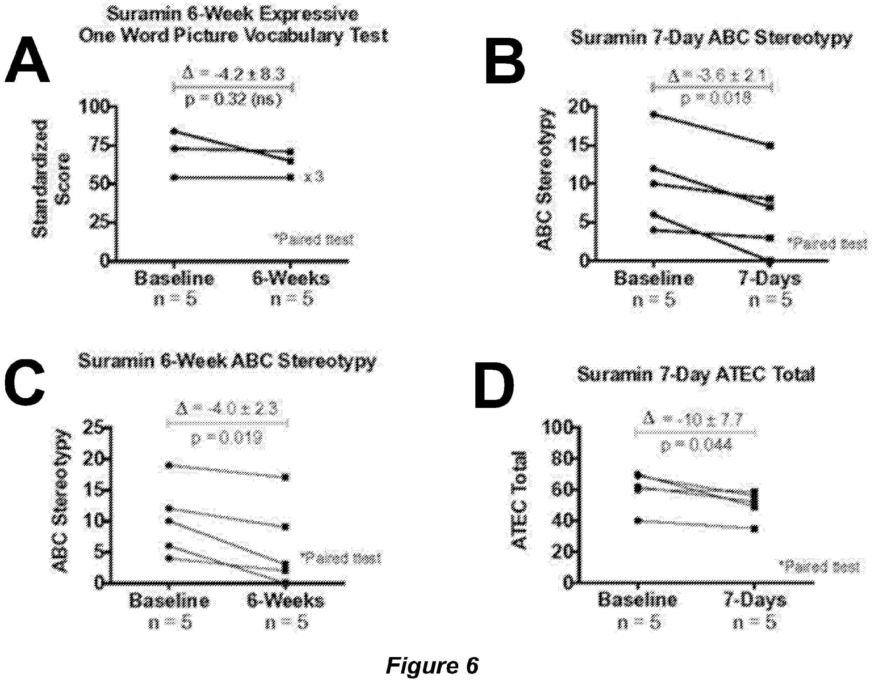

**Fig 6** depicts effects on other endpoints measured in the SAT-1 trial. (A) shows no change in 6-week Expressive One Word Picture Vocabulary scores, (B) shows 7-Day improvement in ABC stereotypy scores after suramin, (C) shows 6-week Improvement in ABC stereotypy scores after suramin, (D) shows 7-Day Improvement in ATEC total scores after suramin.

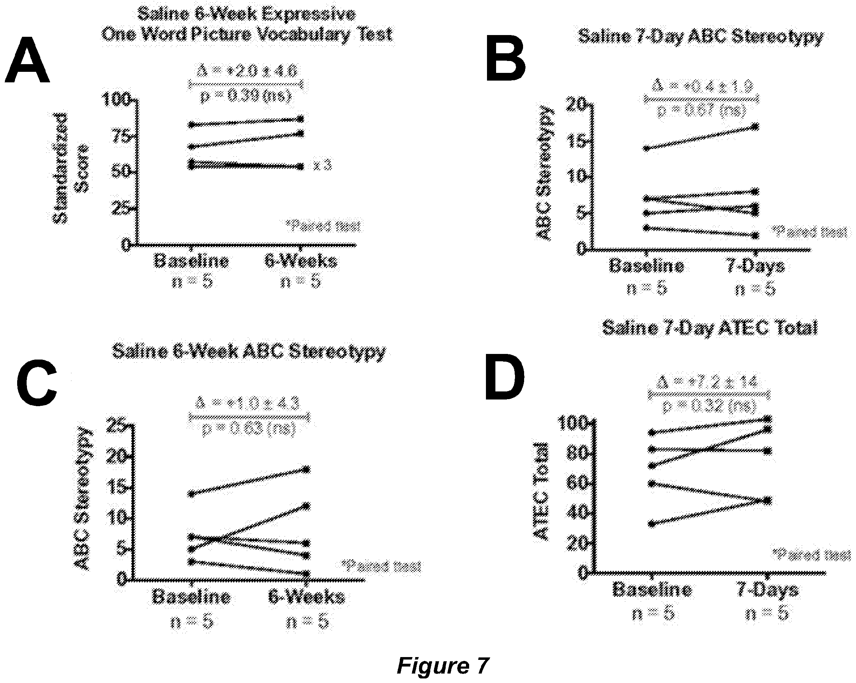

**Fig. 7** depicts effects on more alternative endpoints measured in the SAT-1 trial. (A) shows no change in 6-week EOWPVT scores after saline, (B) shows no change in 7-day ABC stereotypy scores after saline, (C) shows no change in 6-week ABC stereotypy scores after saline, and(D) shows no change in 7-day ATEC total scores after saline.

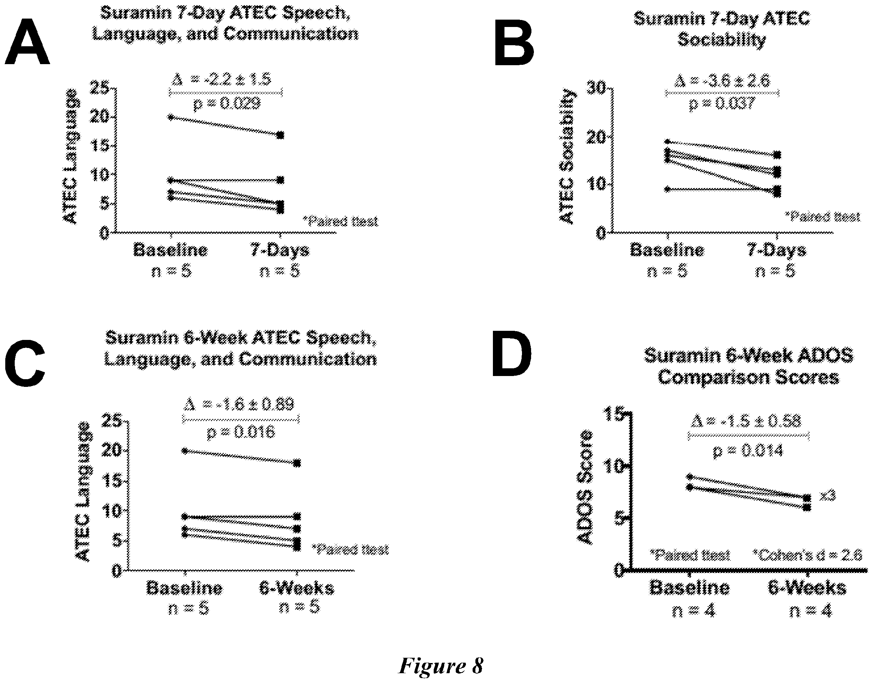

**Fig. 8** depicts effects on ATEC endpoints and ADOS reanalysis after removing a patient who missed a visit. (A) shows improved ATEC speech, language, and communication scores 7-days after suramin, (B) shows improved ATEC sociability scores 7-days after suramin, (C) shows improved ATEC speech, language, and communication scores 6-weeks after suramin, and (D) shows improved ADOS comparison scores after dropping a subject who missed the 6-week visit (N = 4).

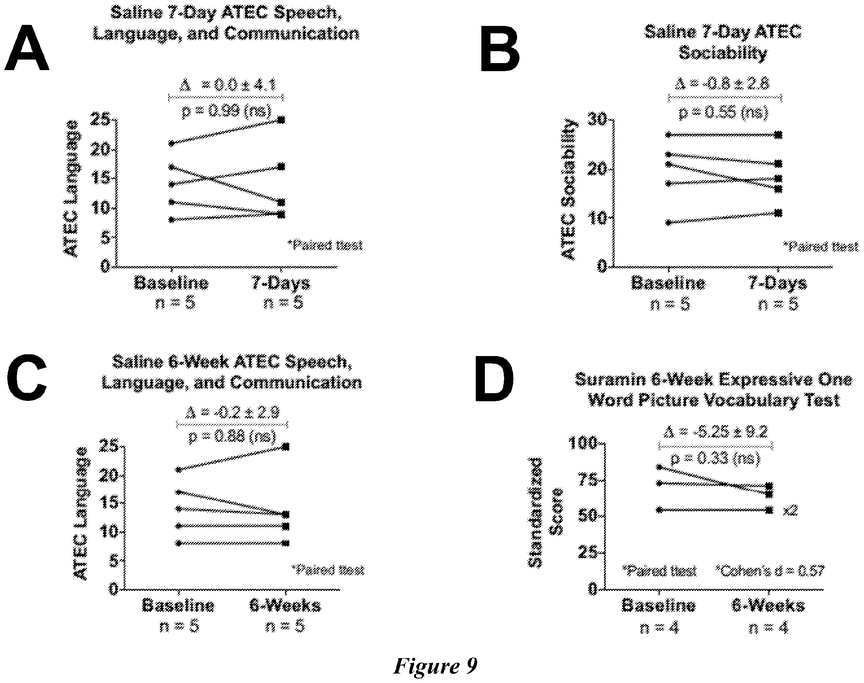

**Fig. 9** depicts effects on more alternative endpoints measured in the SAT-1 trial. (A) shows no change in 7-day ATEC speech, language, and communication after saline, (B) shows no change in 7-day ATEC sociability after saline, (C) shows no change in 6-week ATEC speech, language, and communication scores 6-weeks after saline, and (D) shows no change in EOWPVT scores after dropping subject who missed the 6-week visit (N = 4).

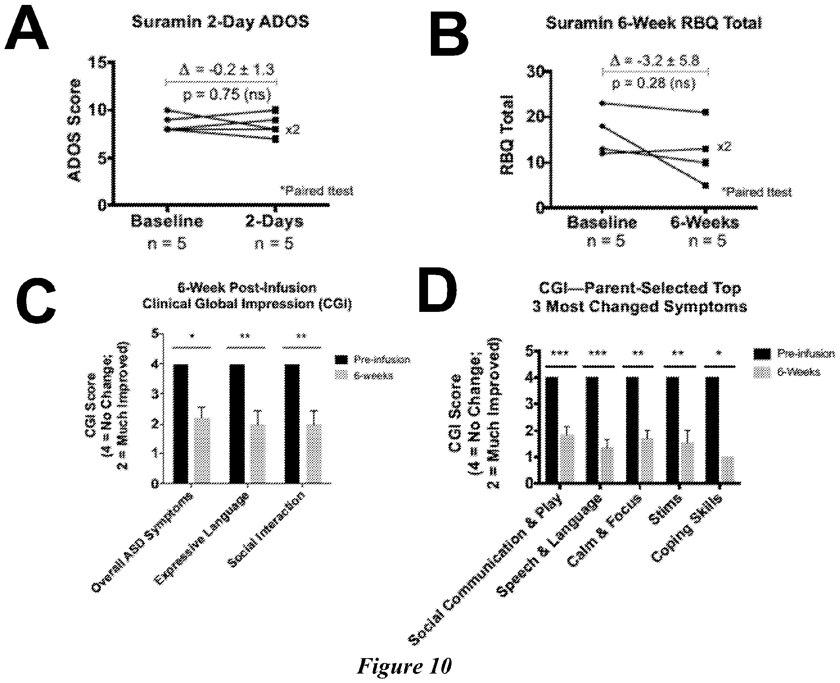

**Fig. 10** depicts effects on more alternative endpoints measured in the SAT-1 trial. (A) shows no change in 2-day ADOS scores after suramin, (B) shows no change in 6-week RBQ total scores after suramin, (C) shows improved core symptoms of ASD and other behaviors by CGI at 6-weeks after suramin. P values: * = 0.05; ** = 0.01; *** = 0.001, and (D) shows top 3, most-changed symptoms named by parents in the 6-week CGI,

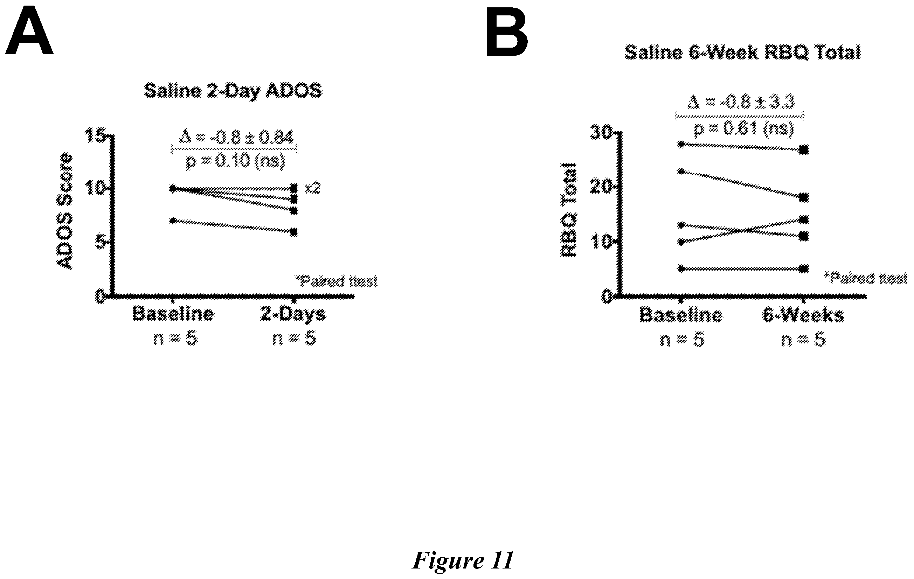

**Fig. 11** depicts effects on final endpoints measured in the SAT-1 trial. (A) shows no change in 2-day ADOS scores after saline and (B) shows no change in 6-week RBQ total scores after saline.

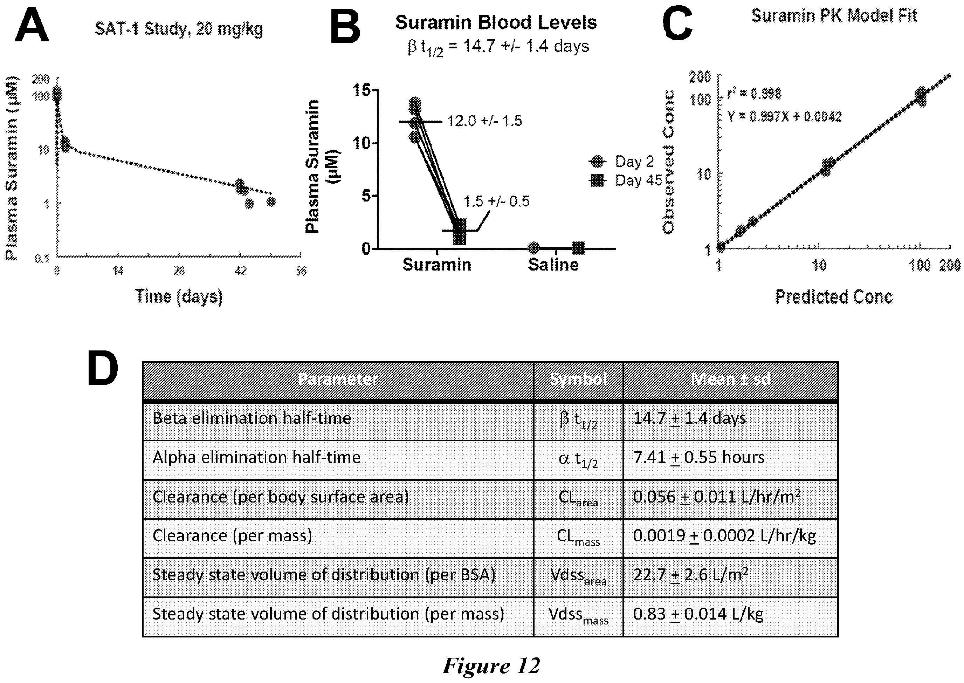

**Fig. 12** depicts pharmacokinetics of suramin in the trial patient population. In (A) 2-compartment model of suramin blood concentrations was fit to the concentration data. The first 48 hours were dominated by the distribution phase. Over 90% of the model is described by the elimination phase. In (B) Plasma suramin concentrations over the elimination phase are depicted. In (C) a goodness-of-fit analysis was performed on the 2-compartment model of suramin blood concentration. The 2-compartment model correlated well with measured values (D) Pediatric PK parameters of suramin (R²=0.998).

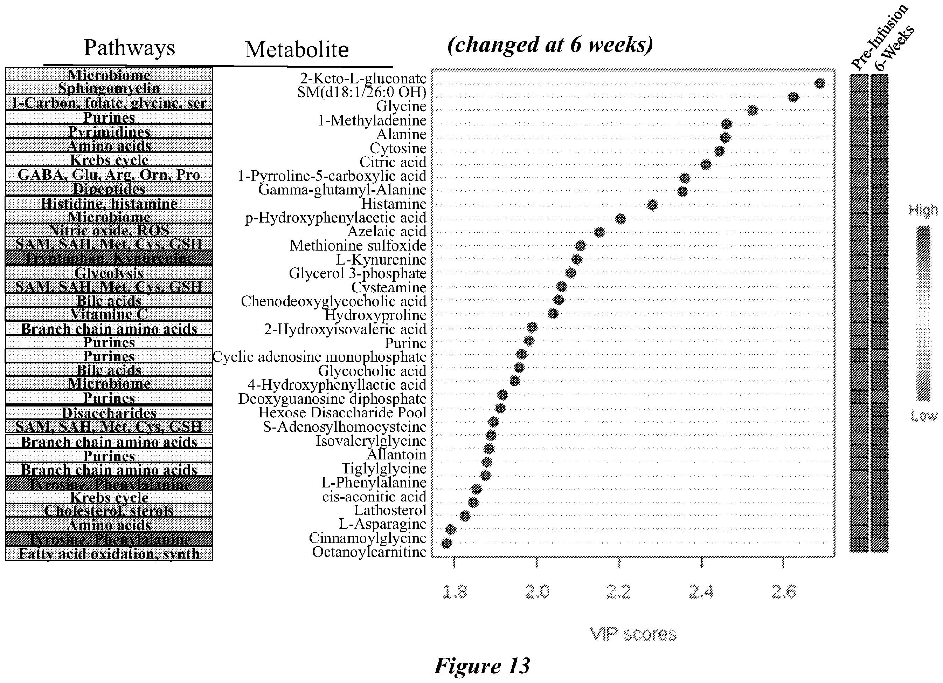

**Fig. 13** depicts suramin pharmacometabolomics at a late time point. Metabolites and pathways changed at 6-weeks are shown according to VIP score.

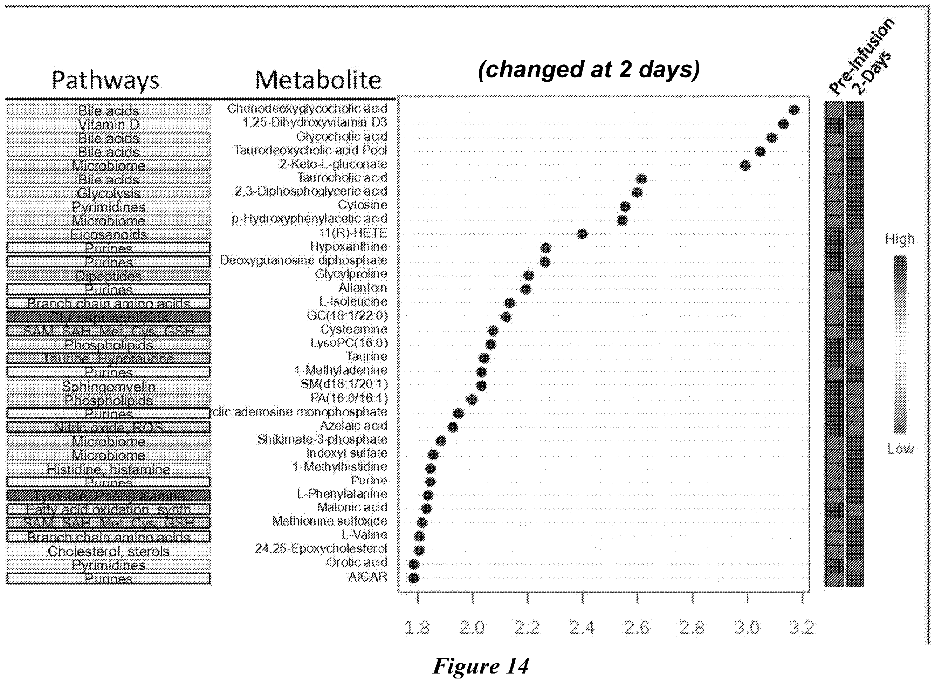

**Fig. 14** depicts suramin pharmacometabolomics at an early time point. Metabolites and pathways changed at 2-days are shown according to VIP score.

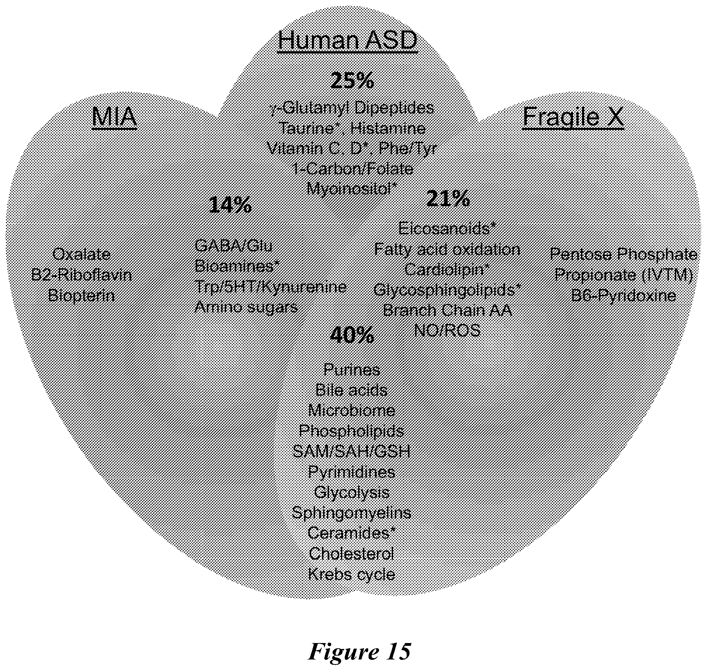

**Fig. 15** depicts a Venn-type diagram showing overlap between metabolite classes upregulated in this study (Human ASD) and metabolite classes upregulated in previous studies using maternal immune activation (MIA) and Fragile X mouse models.

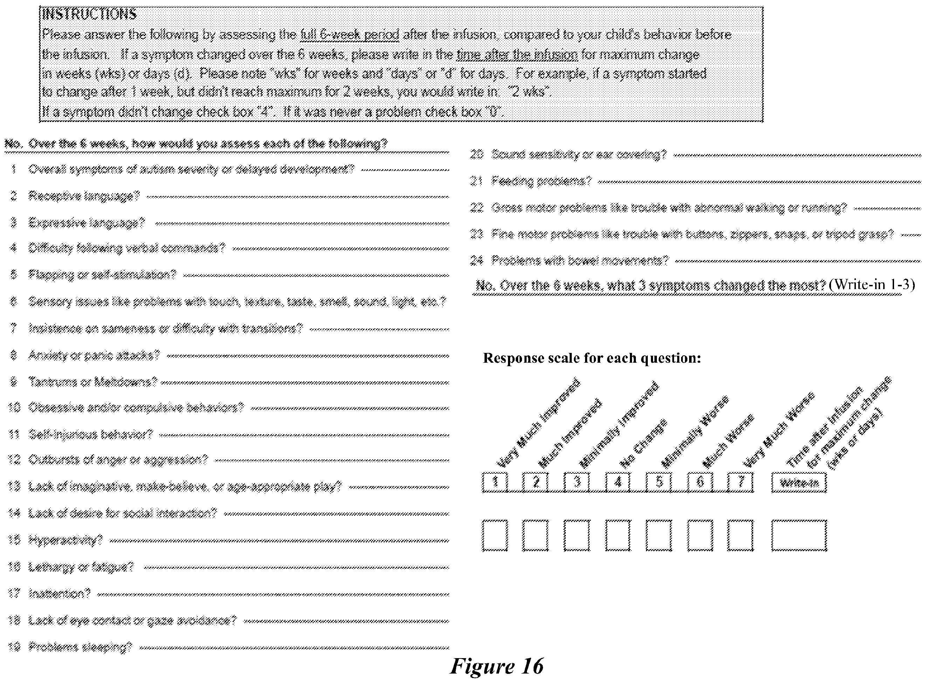

**Fig. 16** depicts an exemplary Clinical Global Impression questionnaire administered in the SAT-1 trial.

### DETAILED DESCRIPTION OF THE INVENTION

Before the embodiments of the invention are described, it is to be understood that such embodiments are provided by way of example only, and that various alternatives to the embodiments of the invention described herein may be employed in practicing the invention. Numerous variations, changes, and substitutions will now occur to those skilled in the art without departing from the invention. Rather, the invention is defined in the claims.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the patent specification, including definitions, will control. In addition, the materials, methods, and examples are illustrative only and not intended to be limiting. Numerous variations, changes, and substitutions will now occur to those skilled in the art without departing from the invention, which is defined by the claims.

Described herein are methods, compositions, and techniques for pharmacological treatment of autism spectrum disorders using antipurinergic agents.

### 1. General Terms

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal and intrasternal injection and infusion.

The phrase "pharmaceutically acceptable" is employed herein to refer to those compounds, excipients, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "pharmaceutically acceptable excipient" or "pharmaceutically acceptable carrier" as used herein means a pharmaceutically acceptable material, composition or vehicle, such as a liquid or solid filler, diluent, excipient, solvent or encapsulating material. Each carrier must be "acceptable" in the sense of being compatible with the other ingredients of the formulation and not injurious to the patient. Some examples of materials which can serve as pharmaceutically acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations.

In some embodiments, the term "prevent" or "preventing" as related to a disease or disorder may refer to a compound that, in a statistical sample, reduces the occurrence of the disorder or condition in the treated sample relative to an untreated control sample, or delays the onset or reduces the severity of one or more symptoms of the disorder or condition relative to the untreated control sample.

The terms "treat," "treating" or "treatment," as used herein, may include alleviating, abating or ameliorating a disease or condition symptoms, preventing additional symptoms, ameliorating or preventing the underlying causes of symptoms, inhibiting the disease or condition, e.g., arresting the development of the disease or condition, relieving the disease or condition, causing regression of the disease or condition, relieving a condition caused by the disease or condition, or stopping the symptoms of the disease or condition either prophylactically and/or therapeutically.

### 2. Antipurinergic Agents

In certain aspects, the disclosure provides compounds that inhibit purinergic signaling and compositions and uses thereof. Compounds of the disclosure may be referred to as antipurinergic agents. In some embodiments, an antipurinergic agent is a pharmacological agent, e.g., a small molecule or polypeptide, capable of modulating, e.g., inhibiting or counteracting, a signaling process naturally mediated by extracellular purine nucleotides or nucleosides in bacteria or higher organisms. Exemplary purine nucleotides or nucleosides known to participate in purinergic signaling include adenosine, ATP, ADP, UTP, UDP, and UDP-glucose.

In some embodiments, an antipurinergic agent is an antagonist of one or more extracellular purinergic receptors. Examples of extracellular purinergic receptors include the G protein-coupled P1 and P2Y receptors as well as the ligand-gated ion channel P2X receptors. In humans, the P1 (also known as the "adenosine") receptor family includes four separately encoded receptors A₁, A_{2A}, A_{2B}, and A₃ (encoded by the genes *ADORA1, ADORA2A, ADORA3B,* and *ADORA3,* respectively), and is so named because all of these receptors respond to adenosine. The P2Y receptor family includes twelve separately encoded receptors P2RY₁, P2RY₂, P2RY₄, P2RY₅/LPA₆, P2RY₆, P2RY₈, P2RY₉/LPAR₄/GDPR23, P2RY₁₀, P2RY₁₁, P2RY₁₂, P2RY₁₃, and P2RY₁₄. The P2X (ion-channel) receptor family includes seven separately-encoded receptors P2X₁, P2X₂, P2X₃, P2X4, P2X₅, P2X₆, and P2X₇. The P2 receptor families (P2X and P2Y) are so named because they respond to nucleotides like ATP/ADP, UTP, and UDP.

In some embodiments, an antipurinergic agent is an inhibitor of a nucleoside transporter which transports one or more purine-receptor-activating nucleosides across the cell membrane. In some embodiments, an antipurinergic agent inhibits purinergic signaling by altering extracellular concentrations of endogenous ligands for purinergic receptors. Examples of nucleoside transporters include the concentrative nucleoside transporters SLC28A1, SLC28A2 and SLC28A3 (which are Na+-dependent symporters) and the equilibrative nucleoside transporters ENT1, ENT2, ENT3, and ENT4 (which are Na+-independent passive transporters).

In some embodiments, an antipurinergic agent is an activator or inhibitor of an ectonucleotidase which hydrolyzes one or more purine-receptor-activating nucleotides in the extracellular space. Examples of ectonucleotidases include ectonucleoside triphosphate diphosphohydrolases (E-NTPDases), ectonucelotide pyrophosphatase/phosphodiesterases (E-NPPs), and alkaline phosphatases (APs).

In some embodiments, an antipurinergic agent is an inhibitor of the Pannexin-1 channel (PANX1), which forms hemichannels involved in release of ATP from the cellular cytoplasm to the extracellular space.

In some embodiments, an antipurinergic agent is a small molecule already identified as having antagonist or inhibitory activity against one or more components of the purinergic signaling system. Such agents include, but are not limited to suramin, AZD9056, pyridoxal-phosphate-6-azophenyl-2', 4'-disulfonic acid, clopidogrel, prasugrel, ticlopidine, ticagrelor, cangrelor, and elinogrel or a salt of any one thereof. In some embodiments, the antipurinergic agent is suramin or a salt thereof. In some embodiments, the antipurinergic agent is represented by the Formula: 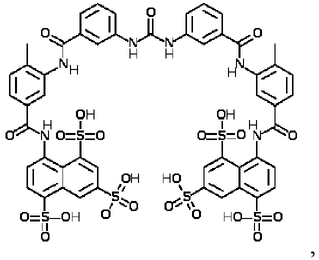 or a salt thereof such as a suramin hexasodium salt. In certain embodiments, the antipurinergic agent is a prodrug of suramin or a salt thereof. In certain embodiments, the antipurinergic agent is not suramin, a prodrug of suramin, or a salt thereof.

In some embodiments, an antipurinergic agent of the disclosure, e.g., suramin, is PEGylated with one or more polyethylene glycol (PEG) groups. In some embodiments, one or more PEG groups are covalently or non-covalently associated with a compound of the disclosure. In some embodiments, a compound of the disclosure is covalently modified with one or more PEG groups. Covalent modification of a compound of the disclosure with one or more PEG groups may reduce dosage frequency, increase drug stability, and enhance protection from proteolytic degradation. In some embodiments, a compound of the disclosure is non-covalently modified with one or more PEG groups, e.g, through hydrogen bonding interactions.

PEG moieties may be prepared by polymerization of ethylene oxide and are commercially available over a wide range of molecular weights from 300 g/mol to 10,000,000 g/mol. PEGs with different molecular weights may have different physical properties, e.g. viscosity, due to chain length effects. PEGs of the disclosure may be selected from branched PEGs, e.g., PEGs that have three to ten PEG chains emanating from a central core group; star PEGs, e.g., PEGs that have 10 to 100 PEG chains emanating from a central core group; and comb PEGs, e.g., PEGs that have multiple PEG chains normally grafted on to a polymer backbone.

PEG groups may be associated with numbers that indicate their average molecular weights, e.g., PEG 400 has a molecular weight of 400 Daltons. Many PEGs include molecules with a distribution of molecular weights, i.e., they are polydisperse. The size distribution can be characterized statistically by its weight average molecular weight (Mw) and its number average molecular weight (Mn), the ratio of which is called the polydispersity index (Mw/Mn). MW and Mn can be measured by mass spectrometry.

In some embodiments, an antipurinergic agent is a molecule discovered via a purinergic receptor assay. An exemplary form of such an assay uses cells in tissue culture ectopically expressing one or more purinergic receptors. Activity of an antagonist on these receptor expressing cells is monitored by the ability of the antagonist to compete away a radiolabeled ligand, e.g., 5'-N-[Adenine-2,8-3H]-Ethylcarboxamidoadenosine in the case of P1 receptors, for the receptor, or by monitoring downstream signaling of the purinergic receptor using calcium imaging reagents, e.g., for the P2X receptors, or downstream production of second messengers such as cAMP, e.g., for theP2Y receptors.

In some embodiments, an antipurinergic agent is a molecule discovered via an extracellular nucleotide release assay. A wide variety of commercial reagents for quantifying extracellular ATP are available; an exemplary class of such reagents are those which detect ATP using recombinant luciferase and a luciferin reagent to convert ATP concentration into a luminescent signal (e.g. the Cell-titer-glo family of reagents available from Promega). Such assays may be used in combination with cultured cells from a variety of different tissue backgrounds expressing various types or subtypes of purinergic receptors, ectonucleotidases, transporters, or channels as described above. In some embodiments, the cells used for these assays are modified to ectopically express one or more of the aforementioned components of purinergic signaling.

Included in the present disclosure are salts, particularly pharmaceutically acceptable salts, of the antipurinergic agents described herein. The compounds of the present disclosure that possess a sufficiently acidic, a sufficiently basic, or both functional groups, can react with any of a number of inorganic bases, and inorganic and organic acids, to form a salt. Alternatively, compounds that are inherently charged, such as those with a quaternary nitrogen, can form a salt with an appropriate counterion, e.g., a halide such as bromide, chloride, or fluoride, particularly bromide.

The compounds described herein may in some cases exist as diastereomers, enantiomers, or other stereoisomeric forms. The compounds presented herein include all diastereomeric, enantiomeric, and epimeric forms as well as the appropriate mixtures thereof. Separation of stereoisomers may be performed by chromatography or by forming diastereomers and separating by recrystallization, or chromatography, or any combination thereof. (Jean Jacques, Andre Collet, Samuel H. Wilen, "Enantiomers, Racemates and Resolutions", John Wiley And Sons, Inc., 1981). Stereoisomers may also be obtained by stereoselective synthesis.

The methods and compositions described herein include the use of amorphous forms as well as crystalline forms (also known as polymorphs). The compounds described herein may be in the form of pharmaceutically acceptable salts. As well, active metabolites of these compounds having the same type of activity are included in the scope of the present disclosure. In addition, the compounds described herein can exist in unsolvated as well as solvated forms with pharmaceutically acceptable solvents such as water, ethanol, and the like. The solvated forms of the compounds presented herein are also considered to be disclosed herein.

In some embodiments, antipurinergic agents, e.g., suramin, may be in the form of a prodrug, e.g., wherein one or more acidic groups in the parent compound is presented as an ester. In some embodiments, or more sulfonic acid moieties on suramin is masked as a prodrug, such as an ester group. The term "prodrug" is intended to encompass compounds which, under physiologic conditions, are converted into pharmaceutical agents of the present disclosure. One method for making a prodrug is to include one or more selected moieties which are hydrolyzed under physiologic conditions to reveal the desired molecule. In other embodiments, the prodrug is converted by an enzymatic activity of the host animal such as specific target cells in the host animal. For example, esters or carbonates, e.g., esters or carbonates of acid groups, are preferred prodrugs of the present disclosure.

Prodrugs are often useful because, in some situations, they may be easier to administer than the parent drug. They may, for instance, be bioavailable by oral administration whereas the parent is not. Prodrugs may help enhance the gastrointestinal absorption of a compound relative to the parent drug. Prodrugs may increase the lipophilicity of an antipurinergic agent relative to the parent drug. In some embodiments, the design of a prodrug increases the effective water solubility of an antipurinergic agent. See, e.g., Fedorak et al., Am. J. Physiol., 269:G210-218 (1995); McLoed et al., Gastroenterol, 106:405-413 (1994); Hochhaus et al., Biomed. Chrom., 6:283-286 (1992); J. Larsen and H. Bundgaard, Int. J. Pharmaceutics, 37, 87 (1987); J. Larsen et al., Int. J. Pharmaceutics, 47, 103 (1988); Sinkula et al., J. Pharm. Sci., 64:181-210 (1975); T. Higuchi and V. Stella, Pro-drugs as Novel Delivery Systems, Vol. 14 of the A.C.S. Symposium Series; and Edward B. Roche, Bioreversible Carriers in Drug Design, American Pharmaceutical Association and Pergamon Press, 1987).

The present disclosure provides methods of producing the compounds of the disclosure. The compounds may be synthesized using conventional techniques. Advantageously, these compounds are conveniently synthesized from readily available starting materials. Synthetic chemistry transformations and methodologies useful in synthesizing the compounds described herein are known in the art and include, for example, those described in R. Larock, Comprehensive Organic Transformations (1989); T. W. Greene and P. G. M. Wuts, Protective Groups in Organic Synthesis, 2d. Ed. (1991); L. Fieser and M. Fieser, Fieser and Fieser's Reagents for Organic Synthesis (1994); and L. Paquette, ed., Encyclopedia of Reagents for Organic Synthesis (1995).

### 3. Compositions of Antipurinergic Agents

In some embodiments, the compounds described herein are formulated into pharmaceutical compositions. In specific embodiments, pharmaceutical compositions are formulated in a conventional manner using one or more physiologically acceptable carriers comprising excipients and auxiliaries which facilitate processing of the active compounds into preparations which can be used pharmaceutically. Proper formulation is dependent upon the route of administration chosen. Any pharmaceutically acceptable techniques, carriers, and excipients are used as suitable to formulate the pharmaceutical compositions described herein: Remington: The Science and Practice of Pharmacy, Nineteenth Ed (Easton, Pa.: Mack Publishing Company, 1995); Hoover, John E., Remington's Pharmaceutical Sciences, Mack Publishing Co., Easton, Pennsylvania 1975; Liberman, H.A. and Lachman, L., Eds., Pharmaceutical Dosage Forms, Marcel Decker, New York, N.Y., 1980; and Pharmaceutical Dosage Forms and Drug Delivery Systems, Seventh Ed. (Lippincott Williams & Wilkins1999).

Pharmaceutical compositions include at least one pharmaceutically acceptable carrier, diluent or excipient and at least one antipurinergic agent, also referred to herein as an active ingredient. The active ingredient is in free-acid or free-base form, or in a pharmaceutically acceptable salt form. In addition, the methods and pharmaceutical compositions described herein include the use of N-oxides, crystalline forms (also known as polymorphs), as well as active metabolites of these compounds having the same type of activity. All tautomers of the compounds described herein are included within the scope of the compounds presented herein. Additionally, the compounds described herein encompass unsolvated as well as solvated forms with pharmaceutically acceptable solvents such as water, ethanol, and the like. The solvated forms of the compounds presented herein are also considered to be disclosed herein. In addition, the pharmaceutical compositions optionally include other medicinal or pharmaceutical agents, carriers, adjuvants, such as preserving, stabilizing, wetting or emulsifying agents, solution promoters, salts for regulating the osmotic pressure, buffers, and/or other therapeutically valuable substances.

Methods for the preparation of compositions comprising the compounds described herein include formulating the compounds with one or more inert, pharmaceutically acceptable excipients or carriers to form a solid, semi-solid or liquid. Solid compositions include, but are not limited to, powders, tablets, dispersible granules, capsules, cachets, and suppositories. Liquid compositions include solutions in which a compound is dissolved, emulsions comprising a compound, or a solution containing liposomes, micelles, or nanoparticles comprising a compound described herein. Semi-solid compositions include, but are not limited to, gels, suspensions and creams. The form of the pharmaceutical compositions described herein include liquid solutions or suspensions, solid forms suitable for solution or suspension in a liquid prior to use, or as emulsions. These compositions also optionally contain minor amounts of nontoxic, auxiliary substances, such as wetting or emulsifying agents, pH buffering agents, and so forth.

In some embodiments, a pharmaceutical composition comprising at least one antipurinergic agent illustratively takes the form of a liquid where the agents are present in solution, in suspension or both. In some embodiments, when the composition is administered as a solution or suspension, a first portion of the agent is present in solution and a second portion of the agent is present in particulate form, e.g., in suspension in a liquid matrix. In some embodiments, a liquid composition includes a gel formulation. In other embodiments, the liquid composition is aqueous.

In some embodiments, aqueous suspensions of the disclosure contain one or more polymers as suspending agents. Polymers may be selected from water-soluble polymers such as cellulosic polymers, e.g., hydroxypropyl methylcellulose, and water-insoluble polymers such as cross-linked carboxyl-containing polymers. Certain pharmaceutical compositions described herein comprise a mucoadhesive polymer, selected for example from carboxymethylcellulose, carbomer (acrylic acid polymer), poly(methylmethacrylate), polyacrylamide, polycarbophil, acrylic acid/butyl acrylate copolymer, sodium alginate and dextran.

Pharmaceutical compositions also, optionally, include solubilizing agents to aid in the solubility of an antipurinergic agent or to facilitate its delivery across a tissue barrier or cellular membrane. The term "solubilizing agent" generally includes agents that result in formation of a micellar solution or a true solution of the agent. Nonionic surfactants that may be used in compositions of the disclosure include, for example, polysorbate 80, glycols, polyglycols, e.g., polyethylene glycol 400, and glycol ethers. Anionic surfactants that may be used in compositions of the disclosure include, for example, alkyl sulfates (such as dioctyl sulfosuccinate) and alkyl ethoxylate sulfates. Cationic surfactants that may be used in compositions of the disclosure include, for example, pyridinium-based compounds such as cetylpyridinium chloride. Zwitterionic surfactants that may be used in compositions of the disclosure include various soy and coconut-based natural products such as cocamidopropyl betaine and lecithin.

Pharmaceutical compositions optionally include one or more pH adjusting agents or buffering agents, including acids such as acetic, boric, citric, lactic, phosphoric and hydrochloric acids; bases such as sodium hydroxide, sodium phosphate, sodium borate, sodium citrate, sodium acetate, sodium lactate and tris-hydroxymethylaminomethane; and buffers such as citrate/dextrose, sodium bicarbonate and ammonium chloride. Such acids, bases and buffers are included in an amount required to maintain pH of the composition in an acceptable range.

Additionally, compositions of the disclosure may also include one or more salts in an amount required to bring osmolality of the composition into an acceptable range. Such salts include those having sodium, potassium or ammonium cations and chloride, citrate, ascorbate, borate, phosphate, bicarbonate, sulfate, thiosulfate or bisulfite anions; suitable salts include sodium chloride, potassium chloride, sodium thiosulfate, sodium bisulfite and ammonium sulfate.

Compositions of the disclosure may also include one or more preservatives to inhibit microbial activity. Suitable preservatives include mercury-containing substances such as merfen and thiomersal; stabilized chlorine dioxide; and quaternary ammonium compounds such as benzalkonium chloride, cetyltrimethylammonium bromide and cetylpyridinium chloride.

Still other useful compositions include one or more surfactants to enhance physical stability or for other purposes. Suitable nonionic surfactants include polyoxyethylene fatty acid glycerides and vegetable oils, e.g., polyoxyethylene (60) hydrogenated castor oil; and polyoxyethylene alkylethers and alkylphenyl ethers, e.g., octoxynol 10, octoxynol 40.

Still other useful compositions include one or more antioxidants to enhance chemical stability where required. Suitable antioxidants include, by way of example only, ascorbic acid and sodium metabisulfite.

In some embodiments, aqueous suspension compositions are packaged in single-dose non-reclosable containers. Alternatively, multiple-dose reclosable containers are used, in which case it is typical to include a preservative in the composition.

In alternative embodiments, other delivery systems for hydrophobic pharmaceutical compounds are employed. Liposomes, emulsions, and microemulsions are examples of delivery vehicles or carriers useful herein. In some embodiments, organic solvents such as N-methylpyrrolidone are also employed. In additional embodiments, the compounds described herein are delivered using a sustained release system, such as semipermeable matrices of solid hydrophobic polymers containing the therapeutic agent. Various sustained release materials are useful herein. In some embodiments, sustained release capsules release the compounds for a few weeks up to over 100 days. Depending on the chemical nature and the biological stability of the therapeutic reagent, additional strategies for protein stabilization are employed.

In some embodiments, the formulations described herein comprise one or more excipients selected from antioxidants, metal chelating agents, thiol containing compounds and other general stabilizing agents. Examples of such stabilizing agents, include, but are not limited to: (a) about 0.5% to about 2% w/v glycerol, (b) about 0.1% to about 1% w/v methionine, (c) about 0.1% to about 2% w/v monothioglycerol, (d) about 1 mM to about 10 mM EDTA, (e) about 0.01% to about 2% w/v ascorbic acid, (f) 0.003% to about 0.02% w/v polysorbate 80, (g) 0.001% to about 0.05% w/v. polysorbate 20, (h) arginine, (i) heparin, (j) dextran sulfate, (k) cyclodextrins, (1) pentosan polysulfate and other heparinoids, (m) divalent cations such as magnesium and zinc; or (n) combinations thereof.

For use in the therapeutic applications described herein, kits and articles of manufacture are also provided. Some such kits comprise a carrier, package, or container that is compartmentalized to receive one or more containers such as vials, tubes, and the like, each of the container(s) comprising one of the separate elements to be used in a method described herein. Suitable containers include, for example, bottles, vials, syringes, and test tubes. The containers are formed from a variety of materials such as glass or plastic.

The articles of manufacture provided herein contain packaging materials. Packaging materials for use in packaging pharmaceutical products include those found in, e.g., U.S. Pat. Nos. 5,323,907, 5,052,558 and 5,033,252. Examples of pharmaceutical packaging materials include, but are not limited to, blister packs, bottles, tubes, inhalers, pumps, bags, vials, containers, syringes, bottles, and any packaging material suitable for a selected formulation and intended mode of administration and treatment. For example, the container(s) includes one or more compounds described herein, optionally in a composition or in combination with another agent as disclosed herein. The container(s) optionally have a sterile access port (for example the container is an intravenous solution bag or a vial having a stopper pierceable by a hypodermic injection needle). Such kits optionally comprising a compound with an identifying description or label or instructions relating to its use in the methods described herein.

A kit of the disclosure may include one or more additional containers, each with one or more of various materials (such as reagents, optionally in concentrated form, and/or devices) desirable from a commercial and user standpoint for use of a compound described herein. Nonlimiting examples of such materials include, but not limited to, buffers, diluents, filters, needles, syringes; carrier, package, container, vial and/or tube labels listing contents and/or instructions for use, and package inserts with instructions for use. A set of instructions will also typically be included. A label is optionally on or associated with the container. For example, a label is on a container when letters, numbers or other characters forming the label are attached, molded or etched into the container itself, a label is associated with a container when it is present within a receptacle or carrier that also holds the container, e.g., as a package insert. In addition, a label is used to indicate that the contents are to be used for a specific therapeutic application. In addition, the label indicates directions for use of the contents, such as in the methods described herein. Optionally the pharmaceutical composition is presented in a pack or dispenser device which contains one or more unit dosage forms containing a compound provided herein. The pack for example contains metal or plastic foil, such as a blister pack. Or, the pack or dispenser device is accompanied by instructions for administration. Or, the pack or dispenser is accompanied with a notice associated with the container in form prescribed by a governmental agency regulating the manufacture, use, or sale of pharmaceuticals, which notice is reflective of approval by the agency of the form of the drug for human or veterinary administration. Such notice, for example, is the labeling approved by the U.S. Food and Drug Administration for prescription drugs, or the approved product insert. For instance, compositions containing a compound provided herein formulated in a compatible pharmaceutical carrier are optionally prepared, placed in an appropriate container, and labeled for treatment of an indicated condition.

### 4. Methods of Treatment

The present invention provides an antipurinergic agent for use in specific methods of treatment, as defined in the claims.

Suitable routes of administration for compositions comprising antipurinergic agents include, but are not limited to, oral, intravenous, rectal, aerosol, parenteral, ophthalmic, pulmonary, transmucosal, transdermal, otic, nasal, and topical administration. In addition, by way of example only, parenteral delivery includes intramuscular, subcutaneous, intravenous, intramedullary injections, as well as intrathecal, direct intraventricular, intraperitoneal, intralymphatic, and intranasal injections.

In some embodiments, a compound as described herein is to be administered in a local rather than systemic manner, for example, via injection of the compound directly into an organ, often in a depot preparation or sustained release formulation. In specific embodiments, long acting formulations are to be administered by implantation (for example subcutaneously or intramuscularly) or by intramuscular injection. Furthermore, in other embodiments, the drug is to be delivered in a targeted drug delivery system, for example, in a liposome coated with organ specific antibody. In such embodiments, the liposomes are targeted to and taken up selectively by the organ. In yet other embodiments, a compound of the disclosure is provided in the form of a rapid release formulation, in the form of an extended release formulation, or in the form of an intermediate release formulation. In yet other embodiments, the compound described herein is to be administered topically.

In certain embodiments, the compositions of the disclosure can also be for delivery as microspheres for slow release in the body. For example, microspheres can be administered via intradermal injection of drug-containing microspheres, which slowly release subcutaneously (see Rao, J. Biomater Sci. Polym. Ed. 7:623-645, 1995; as biodegradable and injectable gel formulations (see, e.g., Gao Pharm. Res. 12:857-863, 1995); or, as microspheres for oral administration (see, e.g., Eyles, J. Pharm. Pharmacol. 49:669-674, 1997). Both transdermal and intradermal routes afford constant delivery for weeks or months.

In some embodiments, the pharmaceutical compositions are formulated in a form suitable for parenteral injection as sterile suspensions, solutions or emulsions in oily or aqueous vehicles. Parenteral injection formulations optionally contain formulatory agents such as suspending, stabilizing and/or dispersing agents. In specific embodiments, pharmaceutical formulations for parenteral administration include aqueous solutions of the active compounds in water soluble form. In additional embodiments, suspensions of the active compounds, e.g., antipurinergic agents are prepared as oily injection suspensions. Suitable lipophilic solvents or vehicles for use in the pharmaceutical compositions described herein include, by way of example only, fatty oils such as sesame oil, or synthetic fatty acid esters, such as ethyl oleate or triglycerides, or liposomes. In some embodiments, aqueous injection suspensions contain substances which increase the viscosity of the suspension, such as sodium carboxymethyl cellulose, sorbitol, or dextran. Optionally, the suspension contains suitable stabilizers or agents which increase the solubility of the compounds to allow for the preparation of highly concentrated solutions. In some embodiments, the active ingredient is in powder form for constitution with a suitable vehicle, e.g., sterile pyrogen-free water, before use.

In still other embodiments, the antipurinergic agents are formulated in rectal compositions such as enemas, rectal gels, rectal foams, rectal aerosols, suppositories, jelly suppositories, or retention enemas, containing conventional suppository bases such as cocoa butter or other glycerides, as well as synthetic polymers such as polyvinylpyrrolidone, PEG, and the like. In suppository forms of the compositions, a low-melting wax such as, but not limited to, a mixture of fatty acid glycerides, optionally in combination with cocoa butter is first melted.

In further embodiments antipurinergic agents described herein can be for use alone or in combination with appropriate additives to make oral tablets, powders, granules or capsules, and if desired, with diluents, buffering agents, moistening agents, preservatives, coloring agents, and flavoring agents. The pharmaceutical agents may be formulated with a buffering agent to provide for protection of the compound from low pH of the gastric environment and/or an enteric coating. A pharmaceutical agent included in a pharmaceutical composition may be formulated for oral delivery with a flavoring agent, e.g., in a liquid, solid or semi-solid formulation and/or with an enteric coating.

In some embodiments, pharmaceutical preparations for oral use are obtained by mixing one or more solid excipient with one or more of the compounds described herein, optionally grinding the resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as: for example, maize starch, wheat starch, rice starch, potato starch, gelatin, gum tragacanth, methylcellulose, microcrystalline cellulose, hydroxypropylmethylcellulose, sodium carboxymethylcellulose; or others such as: polyvinylpyrrolidone (PVP or povidone) or calcium phosphate. In certain embodiments, disintegrating agents are optionally added. Disintegrating agents include, by way of example only, cross linked croscarmellose sodium, polyvinylpyrrolidone, agar, or alginic acid or a salt thereof such as sodium alginate.

In some embodiments, dosage forms, such as dragee cores and tablets, are provided with one or more suitable coating. In specific embodiments, concentrated sugar solutions are used for coating the dosage form. The sugar solutions optionally contain additional components, such as by way of example only, gum arabic, talc, polyvinylpyrrolidone, carbopol gel, polyethylene glycol, and/or titanium dioxide, lacquer solutions, and suitable organic solvents or solvent mixtures. Dyestuffs and/or pigments are also optionally added to the coatings for identification purposes. Additionally, the dyestuffs and/or pigments are optionally utilized to characterize different combinations of active compound doses.

In some embodiments, therapeutically effective amounts of at least one of the compounds described herein are formulated into other oral dosage forms. Oral dosage forms include push fit capsules made of gelatin, as well as soft, sealed capsules made of gelatin and a plasticizer, such as glycerol or sorbitol. In specific embodiments, push fit capsules contain the active ingredients in admixture with one or more filler. Fillers include, by way of example only, lactose, binders such as starches, and/or lubricants such as talc or magnesium stearate and, optionally, stabilizers. In other embodiments, soft capsules contain one or more active compound that is dissolved or suspended in a suitable liquid. Suitable liquids include, by way of example only, one or more fatty oil, liquid paraffin, or liquid polyethylene glycol. In addition, stabilizers are optionally added.

In some embodiments, the pharmaceutical compositions comprising a pharmaceutical agent are formulated for transdermal, intradermal, or topical administration. The compositions can be for administration using a syringe, bandage, transdermal patch, insert, or syringe-like applicator, as a powder/talc or other solid, liquid, spray, aerosol, ointment, foam, cream, gel, paste. This preferably is in the form of a controlled release formulation or sustained release formulation administered topically or injected directly into the skin adjacent to or within the area to be treated, e.g., intradermally or subcutaneously. The active compositions can also be for delivery via iontophoresis. Preservatives can be used to prevent the growth of fungi and other microorganisms. Suitable preservatives include, but are not limited to, benzoic acid, butylparaben, ethyl paraben, methyl paraben, propylparaben, sodium benzoate, sodium propionate, benzalkonium chloride, benzethonium chloride, benzyl alcohol, cetypyridinium chloride, chlorobutanol, phenol, phenylethyl alcohol, thimerosal, and combinations thereof.

An insert, transdermal patch, bandage or article can comprise a mixture or coating of polymers that provide for release of the pharmaceutical agents at a constant rate over a prolonged period of time. In some embodiments, the article, transdermal patch or insert comprises water-soluble pore forming agents, such as polyethylene glycol (PEG) that can be mixed with water insoluble polymers to increase the durability of the insert and to prolong the release of the active ingredients.

Transdermal devices (inserts, patches, bandages) may also comprise a water insoluble polymer. Rate controlling polymers may be useful for administration to sites where pH change can be used to effect release. These rate controlling polymers can be applied using a continuous coating film during the process of spraying and drying with the active compound. In one embodiment, the coating formulation is used to coat pellets comprising the active ingredients that are compressed to form a solid, biodegradable insert.

In certain embodiments, a therapeutically effective amount of at least one of the compounds described herein, e.g., suramin, is to be administered by transdermal iontophoresis or acoustic microcavitation methods. Iontophoresis is a process of transdermal drug delivery by use of a voltage gradient on the skin. A molecule, such as an antipurinergic agent, e.g., suramin, may be transported across the stratum corneum by electrophoresis and electroosmosis and the electric field may also increase the permeability of the skin and produce transient pores in cells.

In acoustic microcavitation, microbubbles are used as vehicles to encapsulate a therapeutic agent which in combination with diagnostic ultrasound permits local targeting and release of the therapeutic agent. The motion of the microbubbles has been shown to increase permeability of both individual cell membranes and the endothelium, thus enhancing therapeutic uptake. In certain embodiments, purinergic agents of the disclosure, e.g., suramin, may be for administration by acoustic microcavitation.

In some embodiments, a condition or disorder meriting treatment according to the invention comprises any neurodevelopmental disorder in a patient meeting the official Diagnostic and Statistical Manual of Mental Disorders version 5 (DSM-5, 2013) criteria for autism spectrum. The DSM-5 describes such disorders as being characterized by, for example: a) persistent deficits in social interaction across multiple contexts, e.g. deficits in social-emotional reciprocity, deficits in nonverbal communicative behaviors, or deficits in developing, maintaining, or understanding relationships; b) restricted, repetitive patterns of behavior, interest or activity; c) presentation of symptoms in the early developmental period; d) significant impairment in social or occupational functioning as a result of symptoms; and e) levels of social communication below that expected for the patient's general developmental level.

The methods of the disclosure may additionally treat conditions or disorders other than autism spectrum disorders. For instance, methods of the disclosure may be used to treat cognitive developmental delay. Cognitive developmental delay may be broadly defined as a significant lag in a child's cognitive development when compared to standardized milestones. Types of cognitive delay include problems with language or speech, vision, movement/motor skills, social and emotional skills, and thinking. A cognitive developmental delay may optionally be the result of a genetic defect, such as Down syndrome, fetal alcohol syndrome, caused by a mother drinking alcohol during pregnancy, fragile X syndrome, an inherited type of cognitive impairment, severe medical problems development soon after birth, often associated with prematurity, or other unknown causes.

The methods of the disclosure may additionally treat intellectual developmental disorder, language disorder, speech sound disorder, social (pragmatic) communication disorder, stereotypic movement disorder, Tourette's disorder, persistent (chronic) motor or vocal tic disorder, other specified or unspecified neurodevelopmental disorders.

In some embodiments, an autism spectrum disorder comprises one or more of the previously used DSM-IV-TR diagnostic categories of autism, Asperger syndrome, pervasive developmental disorder not otherwise specified (PDD-NOS), and childhood disintegrative disorder.

In some embodiments, a patient with an autism spectrum disorder additionally has a known genetic lesion associated with autism spectrum disorders. Such genetic lesions include cytogenetically visible chromosomal abnormalities, copy number variants (CNVs), and DNA mutations. In some embodiments the genetic lesion is associated with another named neurodevelopmental disorder such as Prader-Willi syndrome, Angelman syndrome, Down syndrome, and Turner syndrome. The aforementioned genetic lesions can be identified in patients by a variety of well-known techniques including microscopic karyotyping, fluorescence in situ hybridization (FISH), or array comparative genomic hybridization (aCGH). Curated databases of chromosomal abnormalities reported in autism have been constructed, see for e.g. Marshall et al. Am J Hum Genet 2008; 82:477-488.

In some embodiments, a subject of the treatment methods described herein does not have a known genetic lesion associated with autism spectrum disorders. For example, a subject of the treatment methods described herein may be selected from subjects that do not have cytogenetically visible chromosomal abnormalities, copy number variants (CNVs), or DNA mutations.

In certain embodiments, a subject with an autism spectrum disorder of the treatment methods described herein has a gastrointestinal disease or disorder. In certain embodiments, a subject of the treatment methods described herein has chronic constipation, diarrhea, gastroesophageal reflux disease, or irritable bowel syndrome. In certain embodiments, a subject with an autism spectrum disorder of the treatment methods described herein has a microbiome imbalance. There is accordingly disclosed herein a method for selecting a subject who would benefit from the autism spectrum disorder therapies described herein. In particular, a subject may be selected from a subject with an autism spectrum disorder wherein the subject suffers from one or more symptoms associated with a gastrointestinal disease or disorder.

A patient in need of treatment for autism spectrum disorder according to the invention may be any patient with an autism spectrum disorder that is at a pre-adult neurodevelopmental stage. In some embodiments, a patient in need of treatment for autism spectrum disorder is less than 21 years old. In some embodiments, a patient in need of treatment for autism spectrum disorder is less than 18 years old. In some embodiments, a patient in need of treatment for autism spectrum disorder is less than 16 years old. In other embodiments, a patient in need of treatment for autism spectrum disorder is a child. In yet other embodiments, a patient in need of treatment for autism spectrum disorder is an infant. In some embodiments, a patient in need of treatment for autism spectrum disorder is between 14 months and 16 years old.

Disclosed herein are methods of evaluating the efficacy of the treatment and modulating a treatment regimen where appropriate. A variety of standardized evaluation schemes are available for monitoring the course, severity, and spectrum of functional impairments in patients with autism spectrum disorder or suspected to be at risk for autism-spectrum disorder. Such schemes also may be used to assess the evolution of autism symptoms over time or in response to treatment. Of these, the Autism Diagnostic Observation Schedule (ADOS-2, in its most current iteration, described in Gotham et al. J Autism Dev Disord. 2007 Apr;37(4):613-27.) is uniquely useful for patients of wide age ranges as it has a variety of modules that account for the developmental level and age of the patient. It includes a standardized administration of interactive activities introduced by the examiner which are designed to elicit social interactions, communication, and repetitive behaviors for the purpose of diagnosing an autism spectrum disorder, with procedures optimized for patients from less than 48 months through adulthood. Also useful for evaluating communication impairment in autism spectrum disorder is the Expressive One Word Picture Vocabulary Test (EOWPVT), which assesses verbal expression and the ability to name and generate words (described in Chapman et al. Early Hum Dev. 2015 Jun; 91(6): 373-379.) Additional metrics that may be used to gauge improvement of ASD patients include the caregiver-administered Aberrant Behavior Checklist (ABC, see for e.g. Kaat et al. J Autism Dev Disord. 2014 May;44(5):1103-16.) and Autism Treatment Evaluation Checklist (ATEC, see for e.g. Geier et al. Mental Health Research in Intellectual Disabilities 2013; 6: 255-67). Additionally, a modified version of the Clinical Global Impressions scale (see for e.g. **Fig. 16**) may be used to judge patient progress.

Subjects may generally be monitored for therapeutic effectiveness using assays and methods suitable for the condition being treated, which assays will be familiar to those having ordinary skill in the art and are described herein. Pharmacokinetics of a pharmaceutical agent, or one or more metabolites thereof, that is administered to a subject may be monitored by determining the level of the pharmaceutical agent or metabolite in a biological fluid, for example, in the blood, blood fraction, e.g., serum, and/or in the urine, and/or other biological sample or biological tissue from the subject. Any method practiced in the art and described herein to detect the agent may be used to measure the level of the pharmaceutical agent or metabolite during a treatment course.

In some embodiments, subjects are monitored for therapeutic effectiveness using assays that follow the concentration of one or more non-drug metabolites in patient serum, sputum, blood, or urine as biomarkers of treatment efficacy. Such metabolites can be concentration assayed by a variety of techniques, including but not limited to the LC-MS/MS analysis as in **Example 4**. The metabolites tracked may be early (∼2d) biomarkers of treatment efficacy that are identified in **Table 7,** or may participate in pathways identified in **Table 6.** In particular embodiments, the metabolites tracked as a biomarker of treatment efficacy are one or more chemical entities from **Table 7.** In particular embodiments, the metabolites tracked as a biomarker of treatment efficacy are 5 or more, 10 or more, or 15 or more chemical entities from **Table 6.** The metabolites tracked may be late (∼45d) biomarkers of treatment efficacy that are identified in **Table 8,** or may participate in pathways identified in **Table 5.** In particular embodiments, the metabolites tracked as a biomarker of treatment efficacy are 5 or more, 10 or more, or 15 or more chemical entities from **Table 8.**

In some embodiments, treating an autism spectrum disorder comprises improving one or more symptoms relative to symptoms of said subject prior to said administration, wherein the symptoms are selected from difficulty communicating, difficulty interacting with others, and repetitive behaviors. In some embodiments, treating an autism spectrum disorder comprises improving an assessment score of said subject selected from ADOS, ABC, ATEC, and CGI scores relative to a score prior to said administration. In some embodiments, an assessment score of a subject is improved by about 5% or more, about 10% or more, such as about 15% or more, such as about 20% or more, such as about 25% or more, such as about 30% or more, such as about 35% or more, such as about 40% or more, such as about 45% or more, such as about 50% or more, such as about 55% or more, such as about 60% or more, such as about 65% or more, such as about 70% or more, such as about 75% or more, such as about 80% or more, such as about 85% or more, or even about 90% or more relative to the pre-treatment assessment score.

In some embodiments, the treatment of an autism spectrum disorder may be evaluated by any of the methods described in U.S. Patent No. 9,443,205.

In some embodiments, the methods for treating an autism spectrum disorder described herein comprise improving an ADOS score of a subject by about 1.0 or more, about 1.1 or more, about 1.2 or more, about 1.3 or more, about 1.4 or more, about 1.5 or more, about 1.6 or more, about 1.7 or more, about 1.8 or more, about 1.9 or more, or about 2.0 or more relative to a score of the subject prior to said administration

In some embodiments, the improvement of an assessment score such as ADOS, ABC, ATEC, and CGI, has a p-value of 0.05 or less, about 0.04 or less, about 0.03 or less, or about 0.02 or less. In some embodiments, the effect size of a treatment method described here is about 1 or more, about 1.5 or more, about 2 or more, about 2.5 or more, about 2.6 or more, about 2.7 or more, about 2.8 or more, about 2.9 or more or about 3.0 or more.

The compositions containing the antipurinergic agents described herein can be administered for prophylactic and/or therapeutic treatments. In therapeutic applications, the compositions are to be administered to a patient already suffering from a disease or condition, in an amount sufficient to cure or at least partially arrest the symptoms of the disease or condition. Amounts effective for this use will depend on the severity and course of the disease or condition, previous therapy, the patient's health status, weight, and response to the drugs, and the judgment of the treating physician.

In prophylactic applications, compositions containing the compounds described herein are to be administered to a patient susceptible to or otherwise at risk of a particular disease, disorder or condition. Such an amount is defined to be a "prophylactically effective amount or dose." In this use, the precise amounts also depend on the patient's state of health, weight, and the like. When used in a patient, effective amounts for this use will depend on the severity and course of the disease, disorder or condition, previous therapy, the patient's health status and response to the drugs, and the judgment of the treating physician.

In the case wherein the patient's condition does not improve, upon the doctor's discretion the administration of the compounds may be administered chronically, that is, for an extended period of time, including throughout the duration of the patient's life in order to ameliorate or otherwise control or limit the symptoms of the patient's disease or condition.

In the case wherein the patient's status does improve, upon the doctor's discretion the administration of the compounds may be given continuously; alternatively, the dose of drug being administered may be temporarily reduced or temporarily suspended. In some embodiments, temporary suspension of treatment with an agent of the disclosure is referred to as a "treatment holiday". During a treatment holiday, the agent of the disclosure is not administered to the subject; however, the drug may still be present and detectable in the subject's blood stream for the full length of the holiday or for a portion thereof. In some embodiments, the length of the treatment holiday can vary between 2 days and 1 year, including by way of example only, 2 days, 3 days, 4 days, 5 days, 6 days, 7 days, 10 days, 12 days, 15 days, 20 days, 28 days, 35 days, 50 days, 70 days, or 100 days. In some embodiments, the patient is to be administered at least two doses of an antipurinergic agent of the disclosure, wherein there is a treatment holiday between said doses of about 1 day to about 90 days, such as about 1 day to about 60 days, such as about 1 day to about 30 days, such as about 1 day to about 40 days, such as about 5 days to about 30 days. In some embodiments, the patient is to be administered at least three doses of an antipurinergic agent of the disclosure, wherein there is a treatment holiday between second and third doses of about 10 days to about 90 days, such as about 10 days to about 60 days, such as about 10 days to about 30 days, such as about 10 days to about 40 days. In some embodiments, the third dose is smaller than the second dose. In some embodiments, the treatment holiday between the second and third doses is longer than the treatment holiday between the first and second doses.

In some embodiments, once improvement of the patient's conditions has occurred, a maintenance dose may be administered if necessary. For a maintenance dose, the dosage or the frequency of administration, or both, can be reduced relative to an initial treatment regimen, as a function of the symptoms, to a level at which the improved disease, disorder or condition is retained. Patients may, however, require intermittent treatment on a long-term basis upon any recurrence of symptoms.

The compounds according to the invention may be for administration at a dose selected from about 0.01 mg/kg to 100 mg/kg. For example, an antipurinergic agent of the disclosure may be to be administered in a dose from about 10-30 mg/kg. In some embodiments, a dosage of antipurinergic agent is selected from about 5-50 mg/kg, about 5-45 mg/kg, about 5-40 mg/kg, about 5-35 mg/kg, about 5-30 mg/kg, about 5-25 mg/kg, about 5-20 mg/kg, about 10-50 mg/kg, about 10-45 mg/kg, about 10-40 mg/kg, about 10-35 mg/kg, about 10-30 mg/kg, about 10-25 mg/kg, about 10-20 mg/kg, about 20-50 mg/kg, about 20-45 mg/kg, about 20-40 mg/kg, about 20-35 mg/kg, about 20-30 mg/kg, about 21-50 mg/kg, about 21-45 mg/kg, about 21-40 mg/kg, and about 21-35 mg/kg.

In some embodiments, the dosage of antipurinergic agent for use in the methods described herein is greater than about 20 mg/kg, greater than about 21 mg/kg, greater than about 22 mg/kg, greater than about 23 mg/kg, greater than about 24 mg/kg, greater than about 25 mg/kg, greater than about 26 mg/kg, greater than about 27 mg/kg, greater than about 28 mg/kg, greater than about 29 mg/kg, greater than about 30 mg/kg, or greater than about 40 mg/kg. In some embodiments, the dosage for use in the methods described herein is less than about 80 mg/kg, less than about 70 mg/kg, less than about 60 mg/kg, less than about 50 mg/kg, less than about 40 mg/kg, or less than about 30 mg/kg. In some embodiments, a dose of antipurinergic agent is selected from a combination of any lower and upper limit described in the preceding section. An exemplary dosage is about 500 mg (for a 25 kg subject). The exact dosage may depend upon the route of administration, the form in which the compound is administered, the subject to be treated, the body weight of the subject to be treated, and the experience of the attending physician.

The compounds of the disclosure may be to be administered at a dose selected from about 100 mg/m² to about 700 mg/m². For example, an antipurinergic agent of the disclosure may be to be administered in a dose from about 200 mg/m² to about 600 mg/m². In some embodiments, a dosage of antipurinergic agent is selected from about 250 mg/m² to about 600 mg/m², about 150 mg/m² to about 250 mg/m², about 250 mg/m² to about 350 mg/m², about 350 mg/m² to about 450 mg/m², about 450 mg/m² to about 550 mg/m², about 550 mg/m² to about 650 mg/m², and about 650 mg/m² to about 750 mg/m².

In some embodiments, the dosage of antipurinergic agent for use in the methods described herein is greater than about 100 mg/m², greater than about 150 mg/m², greater than about 200 mg/m², greater than about 250 mg/m², greater than about 300 mg/m², greater than about 350 mg/m², greater than about 400 mg/m², or even greater than about 450 mg/m². In some embodiments, the dosage for use in the methods described herein is less than about 800 mg/m², less than about 750 mg/m², less than about 700 mg/m², less than about 650 mg/m², less than about 600 mg/m², less than about 550 mg/m², less than about 500 mg/m², less than about 450 mg/m², less than about 400 mg/m², or less than about 300 mg/m². In some embodiments, a dose of antipurinergic agent is selected from a combination of any lower and upper limit described in this paragraph. An exemplary dosage is about 300 mg/m², about 400 mg/m², or about 500 mg/m². The exact dosage may depend upon the route of administration, the form in which the compound is administered, the subject to be treated, the body surface area of the subject to be treated, and the experience of the attending physician.

In some embodiments, the dose of the compounds according to the disclosure is selected to achieve a desired continuous steady-state plasma concentration or range of steady-state plasma concentrations. Such doses can be readily selected after determining standard pharmacokinetic parameters for the compound such as half-life and volume of distribution (determined by measuring serum concentrations of the compound at various time points after administration). Pharmacokinetic parameters and dosing parameters are then input into a pharmacokinetic model and the model is solved for dose. A variety of such models are described, for example, in Remington Essentials of Pharmaceutics (2013), especially p.705-725.

In some embodiments, a steady-state plasma concentration is to be accomplished by first administering a loading dose of the antipurinergic agent (e.g. suramin or a salt or prodrug thereof) followed by maintenance doses smaller than the loading dose (e.g. 50-80% of the loading dose) at intervals roughly equivalent to the half-life of the antipurinergic agent (for example, the half-life of the antipurinergic agent can be previously determined in a single-dosing experiment such as the one described for **Fig. 12**). By way of example only, the maintenance doses of the antipurinergic agent may be to be administered every 14 days (roughly the half-life determined by single dosing in **Fig. 12**). In some cases, the half-life of the antipurinergic agent is different (e.g. longer) upon administration of more than one dose, in which the half-life upon multiple dosing can be determined, and the interval between maintenance doses can be longer (e.g. greater than 14 days) than the initially determined half-life of the drug in naive patients.

Blood plasma levels of a therapeutic agent of the disclosure, e.g., suramin, administered intravenously often peak following administration, e.g., within 2 days of administration of said therapeutic agent, and this peak value may fall outside of the desired continuous steady-state plasma concentration. In certain embodiments, in determining a continuous steady-state plasma concentration of a therapeutic agent, measurements from the first day, first two days or first three days following administration, e.g. intravenous administration, are excluded from the evaluation of a continuous steady-state. To illustrate, an antipurinergic agent is to be intravenously delivered over multiple doses to a subject in an amount sufficient to maintain the antipurinergic agent plasma levels of said subject within a range of from about 3 µM to about 15 µM for 100 days or more. For the day that the agent is to be administered and the first day following each dose, the antipurinergic agent plasma levels peak and exceed 15 µM but this initial peak is excluded from the continuous steady-state plasma concentration calculation.

In particular embodiments, an antipurinergic agent according to the disclosure, e.g., suramin or a salt thereof, is to be administered to maintain plasma levels of said antipurinergic agents in a target range from about 1 µM to about 100 µM over a period of time, e.g., from about 2 days to about 100 days, from about 20 days to about 180 days, or from about 20 days to about 365 days. In some embodiments, compounds according to the disclosure are to be administered to maintain plasma levels from about 2 µM to about 50 µM over a period of time, e.g., from about 2 days to about 100 days. In other embodiments, antipurinergic agents according to the disclosure are to be administered to maintain plasma levels of said agents from about 1 µM to about 40 µM, about 1 µM to about 35 µM, about 1 µM to about 30 µM, about 1 µM to about 25 µM, about 4 µM to about 25 µM, about 5 µM to about 25 µM, about 1 µM to about 20, about 1 µM to about 15 µM, about 2 µM to about 15 µM, about 3 µM to about 15 µM, about 4 µM to about 15 µM, about 5 µM to about 15 µM, about 6 µM to about 15 µM, about 7 µM to about 15 µM, about 8 µM to about 15 µM, about 9 µM to about 15 µM, such as from about 1 µM to about 15 µM. In some embodiments, compounds according to the disclosure are to be administered to maintain plasma levels from about 3 µM to about 10 µM for about 10 days to about 100 days, about 15 days to about 100 days, about 20 days to about 100 days, about 25 days to about 100 days, about 30 days to about 100 days, about 35 days to about 100 days, or from about 40 days to about 100 days. In some embodiments, compounds according to the disclosure are to be administered to maintain plasma levels from about 5 µM to about 25 µM for about 10 days to about 100 days, about 15 days to about 100 days, about 20 days to about 100 days, about 25 days to about 100 days, about 30 days to about 100 days, about 35 days to about 100 days, or from about 40 days to about 100 days. In certain embodiments, the maintenance of plasma levels for a period of time is to be measured in non-contiguous days, e.g., excludes the day or two immediately following administration of a dose when plasma levels peak. For example, dose 1 is given on day 1, does 2 is given on day 30 and dose 3 on day 60 and the plasma levels are maintained from about 5 µM to about 25 µM for 100 days which excludes the plasma levels on day 1 and 2, day 30 and 31 and day 60 and 61. However, it should be noted that the claims recite that the method comprises feature (a), feature (b) or feature (c) as defined in claim 1.

In some embodiments, antipurinergic agents according to the disclosure are to be administered to maintain plasma levels of said agents at more than about 1 µM, more than about 2 µM, more than about 3 µM, more than about 4 µM, more than about 5 µM, more than about 6 µM, more than about 7 µM, more than about 8 µM, more than about 9 µM, about 10 µM from about 5 µM to about 15 µM, wherein any of the preceding plasma levels is maintained for about 10 days to about 100 days, about 15 days to about 100 days, about 20 days to about 100 days, about 25 days to about 100 days, about 30 days to about 100 days, about 35 days to about 100 days, or from about 40 days to about 100 days. In some embodiments, antipurinergic agents according to the disclosure are to be administered to maintain plasma levels of said agents at less than about 30 µM, less than about 25 µM, less than about 20 µM, less than about 15 µM, less than about 14 µM, less than about 13 µM, less than about 12 µM, less than about 11 µM, less than about 10 µM, or less than about 9 µM, wherein any of the preceding plasma levels is maintained for about 10 days to about 100 days, about 15 days to about 100 days, about 20 days to about 100 days, about 25 days to about 100 days, about 30 days to about 100 days, about 35 days to about 100 days, or from about 40 days to about 100 days. In some embodiments, the disclosure includes maintaining a plasma concentration in a range formed from any of the lower and upper limits described herein. Again, it should be noted that the claims recite that the method comprises feature (a), feature (b) or feature (c) as defined in claim 1.

In some embodiments, antipurinergic agents according to the disclosure are to be administered to maintain any one of the plasma ranges discussed in the preceding sections or a range of plasma concentrations for a period of time, as described in the preceding section, for about 2 days or more, about 3 days or more, about 4 days or more, about 5 days or more, about 7 days or more, about 10 days or more, about 14 days or more, about 15 days or more, about 20 days or more, about 21 days or more, about 25 days or more, about 28 days or more, about 30 days or more, about 35 days or more, about 40 days or more, about 42 days or more about 50 days or more, about 55 days or more, about 60 days or more, about 70 days or more, about 80 days or more, about 90 days or more, or even about 100 days or more. In some embodiments, antipurinergic agents according to the disclosure are to be administered in a manner to maintain a plasma antipurinergic agent range, as described in the preceding section, for about 2 days to about 100 days, such as about 10 days to about 100 days, such as about 20 days to about 100 days, such as about 25 days to about 100 days, such as about 30 days to about 100 days. In some embodiments, the level of antipurinergic agent in plasma may be calculated based on known factors such as characteristics of the antipurinergic agent, patient characteristics, plasma levels measured from previous administrations to a patient, etc. In some embodiments, the level of antipurinergic agent in plasma of a patient to be administered the therapy may be measured one or more times over the course of treatment. Again, it should be noted that the claims recite that the method comprises feature (a), feature (b) or feature (c) as defined in claim 1.

In some embodiments, suramin or a salt thereof is to be administered in an amount such that following administration, the AUC is about 80 µM*days to about 400 µM*days. In certain embodiments, suramin or a salt thereof is to be administered in an amount such that following administration, the AUC is from 300 µM*days to 1700 µM*days from one to six months. In certain embodiments, suramin is to be administered such that the AUC for 180 days of treatment is from about 900 µM*days to about 1700 µM*days, from about 900 µM*days to about 1200 µM*days, from about 1300 µM*days to about 1700 µM*days, or from about 1400 µM*days to about 1600 µM*days. In certain embodiments, over a 180 day treatment cycle, suramin is to be administered from 3 to 7 times, such as from 4 to 6 times such as 5 times. In certain embodiments, over a 180 day treatment cycle, suramin is to be administered once every 25 to 40 days, such as once every 24 to 35 days. In certain embodiments, suramin is to be administered such that the AUC for 365 days or treatment is from about 1500 µM*days to about 4000 µM*days. In certain embodiments, over a 365 day treatment cycle, suramin is to be administered from 6 to 14 times, such as from 8 to 12 times such as 10 times. In certain embodiments, over a 365 day treatment cycle, suramin is to be administered once every 25 to 40 days, such as once every 24 to 35 days.

In some embodiments, suramin or a salt thereof is to be administered in multiple doses such that the blood plasma level of said agent is equivalent to a single intravenous administration of about 10 mg/kg to about 30 mg/kg. In some embodiments, suramin or a salt thereof is to be administered in multiple doses such that the blood plasma level of said agent is equivalent to a single intravenous administration of about 400 mg/m² to about 700 mg/m².

There is disclosed herein a method of treating an autism spectrum disorder in a subject in need thereof, wherein said treatment comprises administering to said subject a dose of an antipurinergic agent of more than 20 mg/kg. Optionally, the antipurinergic agent is administered in a dose of more than 20 mg/kg and in an amount sufficient to maintain serum creatinine levels of said subject below about 2.0 mg/dL, below about 1.9 mg/dL, below about 1.8 mg/dL, below about below about 1.7 mg/dL, below about 1.6 mg/dL, below about 1.5 mg/dL, below about 1.4 mg/dL, below about 1.3 mg/dL, below about 1.2 mg/dL, below about 1.1 mg/dL, or below about 1.0 mg/dL following said administration. Optionally, the antipurinergic agent is administered in a dose of more than 20 mg/kg, more than about 25 mg/kg, or more than about 30 mg/kg, wherein the amount is sufficient to maintain serum creatinine levels of said subject below about 1.3 mg/dL, such as below about 1.0 mg/dL.

Also disclosed herein is a method of treating an autism spectrum disorder in a subject in need thereof, wherein said treatment comprises administering to said subject a dose of an antipurinergic agent of more than 500 mg/m². Optionally, the antipurinergic agent is administered in a dose of more than 500 mg/m² and in an amount sufficient to maintain serum creatinine levels of said subject below about 2.0 mg/dL, below about 1.9 mg/dL, below about 1.8 mg/dL, below about below about 1.7 mg/dL, below about 1.6 mg/dL, below about 1.5 mg/dL, below about 1.4 mg/dL, below about 1.3 mg/dL, below about 1.2 mg/dL, below about 1.1 mg/dL, or below about 1.0 mg/dL following said administration. Optionally, the antipurinergic agent is administered in a dose of more than 500 mg/m², more than about 550 mg/m², or more than about 600 mg/m², wherein the amount is sufficient to maintain serum creatinine levels of said subject below about 1.3 mg/dL, such as below about 1.0 mg/dL.

In some embodiments, an antipurinergic agent of the disclosure is to be administered in a dose of more than 20 mg/kg and in an amount sufficient to maintain serum aminotransferase levels of said subject below about 60 U/L, below about 55 U/L, below about 50 U/L, below about 45 U/L, below about 40 U/L, below about 35 U/L, below about 30 U/L, below about 25 U/L or below about 20 U/L following said administration. In some embodiments, an antipurinergic agent of the disclosure is to be administered in a dose of more than 20 mg/kg, more than about 25 mg/kg, or more than about 30 mg/kg, wherein the amount is sufficient to maintain serum aminotransferase levels of said subject below about 40 U/L.

In some embodiments, an antipurinergic agent of the disclosure is to be administered in a dose of more than 500 mg/m² and in an amount sufficient to maintain serum aminotransferase levels of said subject below about 60 U/L, below about 55 U/L, below about 50 U/L, below about 45 U/L, below about 40 U/L, below about 35 U/L, below about 30 U/L, below about 25 U/L or below about 20 U/L following said administration. In some embodiments, an antipurinergic agent of the disclosure is to be administered in a dose of more than 500 mg/m², more than about 550 mg/m², or more than about 600 mg/m², wherein the amount is sufficient to maintain serum aminotransferase levels of said subject below about 40 U/L.

Also disclosed herein is a method of treating a subject that would benefit from treatment with an antipurinergic agent, comprising identifying a subject with an autism spectrum disorder, wherein the autism spectrum disorder is not a known syndromic form of autism spectrum disorder caused by DNA mutation or chromosomal copy number variation (CNV). Optionally, the method further comprises treating said subject by administering an antipurinergic agent in response to said identifying.

Also disclosed herein is a method of treating a subject that would benefit from treatment with an antipurinergic agent, comprising identifying a subject with an autism spectrum disorder, wherein the autism spectrum disorder is a known syndromic form of autism spectrum disorder caused by DNA mutation or chromosomal copy number variation (CNV). Optionally, the method further comprises treating said subject by administering an antipurinergic agent in response to said identifying.

In some embodiments, antipurinergic agents of the disclosure are to be administered to maintain plasma concentrations for time periods that may necessitate multiple doses of the compound. In some embodiments, antipurinergic agents of the disclosure are to be administered daily, weekly, once every two weeks, once every three weeks, once every four weeks, once every five weeks, once every six weeks, once every seven weeks, or once every eight weeks.

In certain embodiments, antipurinergic agents of the disclosure are to be administered, e.g., intravenously administered, once every two weeks, once every three weeks, once every four weeks, once every five weeks or even once every six weeks. In certain embodiments, an antipurinergic agent of the disclosure is to be administered once every 25 to 40 days over the course of three months or more. For example, an antipurinergic agent of the disclosure may be to be administered once every 25 to 40 days at an amount individually selected at each dose from 250 mg/m² to about 700 mg/m². In certain embodiments, an antipurinergic agent of the disclosure may be to be administered once every 25 to 40 days, such as once every 25 to 35 days. In certain embodiments, the dosing of antipurinergic agent decreases over the course of administration. For example, the first dose to be administered to a subject is selected from 450 mg/m² to about 550 mg/m² and 25 to 40 days later a second dose of a lesser amount is to be administered selected from about 350 mg/m² to about 450 mg/m². In certain embodiments, the monthly doses may be the same for one or more months. For example, a third dose from the preceding example may be an equivalent amount to the second smaller dose, e.g., first dose: 500 mg/m², second dose: 400 mg/m², third dose: 400 mg/m².

In certain embodiments, compounds according to the disclosure are to be administered via a route that is not intravenous, to maximize patient compliance or ease of caregiver administration in home environments. Multiple studies have documented challenges in medication compliance for autism spectrum disorder subject. Particular routes of administration uniquely convenient for treatment of autism patients are oral, subcutaneous, and rectal administration. Other routes of administration convenient for caregiver administration include intramuscularly, vaginally, by inhalation, by nebulization, cutaneously, and transdermally.

### EXAMPLES

The following illustrative examples are representative of embodiments of the methods described herein and are not meant to be limiting in any way.

### General Methods:

### Standardized Testing and Questionnaires

Two observational examinations were performed by a clinician at 3 time points: baseline (56 ± 8 days; mean ± SEM; before the infusion), 2-days post-infusion, and 6-weeks post-infusion. The two examiner-based metrics were the Autism Diagnostic Observation Schedule, 2nd edition (ADOS-2) with video and audio files recorded on 3 cameras, and the Expressive One Word Picture Vocabulary Testing (EOWPVT). Both of these observational metrics were administered by a trained and certified examiner using approved test materials. Three standardized questionnaires were completed by parents at 3 time points: baseline, 7-days post-infusion, and 6-weeks post-infusion. The three standardized questionnaires completed by parents were the 58-question Aberrant Behavior Checklist (ABC), the 75-item Autism Treatment Evaluation Checklist (ATEC), and the 33-item repetitive behavior questionnaire (RBQ). Parents were asked to complete these three instruments with reference to how their child behaved in the previous 7 days. At the end of the six weeks, we included a 24-question Clinical Global Impression (CGI) questionnaire **(****Fig. 16****).** In addition, parents were asked to list the 3 top behaviors or symptoms that they observed to be most changed over the previous 6-weeks. To minimize the misinterpretation of natural day-to-day variations in symptoms, parents were asked to mark a symptom as changed in the 6-week CGI only if it had lasted for at least 1 week.

### Verification of Data Completeness and Transcription Accuracy

Standardized questionnaire responses and the ADOS-2 and EOWPVT scores (5,490 cells of data) were compiled in spreadsheets from the original hard copy forms and from the electronic medical records. A total of 87 cells (1.6%) of the 5,490 outcome scores were either left blank, asked about a symptom that did not apply, or were missing. One participant missed the 6-week ADOS and EOWPVT evaluations because of scheduling difficulties. His 2-day results were used as an estimate of his 6-week scores. ADOS scores remained significant when this subject was dropped from the analysis **(****Fig. 8D****).** EOWPVT results were also unchanged (Fig. 9D). The 4,210 cells of laboratory and vital sign data were also collected and reviewed. When specific cells of data were found to be missing, they were manually confirmed by inspection of the original questionnaire, laboratory results, and clinical data sheets. A random generator program was written that randomly selected 5% of the data. These randomly selected cells of data that were then manually checked for transcription accuracy by reviewing the hard copy responses and Red Cap electronic medical records.

### Clinical Global Impression (CGI)

We developed a 24-question Clinical Global Impression (CGI) instrument designed to assess the core symptoms of autism spectrum disorders and some of the most common comorbid features **(****Fig. 16****).** The CGI instrument scoring system was the traditional 7-point, CGI-Improvement scale. See, for e.g. Busner and Targum. Psychiatry. 2007(4):28-37. In this scale, the historian gives a score of 0 if the symptom "was never a problem", a 1 for "very much improved", a 4 for "no change", and a 7 for "very much worse". In addition to the 24 structured questions, we asked the parents to write in the top 3 symptoms or behaviors that were most changed over the 6 weeks since the suramin infusion. This hybrid design of structured and openended responses permitted us to capture a large number of clinical outcomes associated with single dose suramin treatment.

### Example 1: Phase I/II SAT-1 trial for suramin in autism spectrum disorder

### Inclusion/exclusion criteria:

All children met DSM-5 diagnostic criteria for autism spectrum disorders, and received confirmatory testing by Autism Diagnostic Observation Schedule, 2nd edition (ADOS-2) examination. Inclusion criteria included males, ages 4-17 years, living in the San Diego, California region, with a confirmed diagnosis of ASD.

Exclusion criteria included children who weighed less than the 5th percentile for age, took prescription medications, or had laboratory evidence of liver, kidney, heart, or adrenal abnormalities. Children living more than a 90-minute drive from the testing sites in La Jolla, CA were excluded to eliminate the possibility of aberrant behaviors resulting from extended car travel. Children with known syndromic forms of ASD caused by DNA mutation or chromosomal copy number variation (CNV) were excluded in this first study. Families were asked not to change their children's therapy (e.g., supplements, speech, and behavioral therapies) or diet throughout the study period. Signed informed consent, with additional consent for video and still image photography, was obtained from the parents of all participants before randomization.

### Study Design:

The study was conducted from May 27, 2015 (date of the first child to be enrolled) to March 3, 2016 (date of the last child to complete the study).

20 males with ASD were screened. 16 met entry criteria. 10 participants could be matched by age, non-verbal IQ, and ADOS scores into 5 pairs. The randomization sequence was generated electronically by the biostatistical team. Subjects within each pair were allocated to receive suramin or saline according to the prospectively determined randomization sequence. The randomization sequence was concealed from the clinical team and implemented by the UCSD investigational pharmacy, which prepared drug and placebo for infusion. The design was double-blind. The mask was not broken until all subjects had completed the study and all clinical data had been collected. Characteristics of patients included in each arm of the study are summarized in **Table** 1 and patient selection is summarized in **Fig. 1****.**

Pharmacological treatments were performed by first administering a test dose, followed by a longer infusion after verification of no immediate adverse effects. Immediately before the infusion, height and weight were recorded, vital signs and capillary oxygen saturation (pulse oximetry) measured, physical and neurological examinations were conducted, and urine and blood for safety monitoring, pharmacology, and metabolomics was collected.

For the test dose, 50 mg of suramin hexasodium (Bayer Pharmaceuticals, Inc.) in 5 mL of saline (experimental arm, 5 patients), or 5 mL of saline only (control arm, 5 patients) were given by slow intravenous (IV) push over 3 minutes, followed by a 10 mL flush of saline. One hour after the test dose, vital signs were repeated and a single infusion of either suramin (20 mg/kg, minus the 50 mg test dose, in 50 mL, up to a maximum of 1 gram) or saline (50 mL IV) was given over 30 minutes, followed by a 10 mL flush of saline.

Toxicity, pharmacokinetics and safety monitoring were performed via biological samples (blood and urine) collected at baseline (32 ± 6 days before the infusion; mean ± SEM), immediately before the infusion, 1 hour after the infusion, 2 days after, and 45 days after the infusion. At each of these time points, 18 vital sign and anthropometric features, 19 complete blood count (CBC) parameters, 20 blood chemistry measures, 3 thyroid and cortisol measures, and 5 lipid measures were monitored (see **Fig. 2** **and** **Fig. 3** for an overview of this data). Suramin pharmacokinetics was assessed by serum isolation at the 4 time points immediately before and after the infusion. Additionally, 24 urinalysis features were measured at 4 times: baseline, pre-infusion, 2-days post-infusion, and 45-days post-infusion. Unexpected and adverse events were recorded as they occurred and graded in severity according to the National Cancer Institute Common Terminology Criteria for Adverse Events v4.03 (CTCAE) scale. Additional pharmacovigilence monitoring included daily scripted phone calls in the first week, then 4 weekly calls until the exit examinations at 6 weeks.

Behavioral and neurological effects of treatment were assessed primarily by the examiner-based ADOS-2 protocol scores and standardized vocabulary testing (Expressive One Word Picture Vocabulary Test, EOWPVT), with secondary evaluation via parent-based assessments (Aberrant Behavior Checklist (ABC) scores, Autism Treatment Evaluation Checklist (ATEC) scores, Clinical Global Impression of Improvement (CGI) scores, and Repetitive Behavior Questionnaire (RBQ) scores). See **General Methods** for information related to behavioral test administration. Prior to the study, diagnosis of each of the enrolled participants was confirmed by ADOS-2 comparison scores of ≥ 7, and non-verbal IQ was tested by Leiter-3 examination. Examiner-based outcomes (ADOS and EOWPVT) were assessed at 2-days and 6-weeks after the infusion. Parent-based outcomes (ABC, ATEC, CGI, and RBQ) were assessed at 7-days and 6-weeks after the infusion. To minimize the effects of natural behavioral variability, the parents were instructed to mark a behavior as changed only if it was persistently changed for at least 1 week.

The study was approved the by the US Food and Drug Administration (IND#118212), and the University of California, San Diego (UCSD) Institutional Review Board (IRB Project #150134). The study conformed to the World Medical Association Declaration of Helsinki-Ethical Principles for Medical Research Involving Human Subjects, and the International Council for Harmonization (ICH) E6 Good Clinical Practice (GCP) guidelines. The trial was registered with clinical trials.gov (https://clinicaltrials.gov/ct2/show/NCT02508259). The completion and reporting of the SAT-1 trial is in keeping with CONSORT 2010 guidelines.

**Table 1: Group characteristics for patients in each arm of the study**

| **Parameter** | **Suramin Group Mean ± SD (Range) or Number** | **Placebo Group Mean ± SD (Range) or Number** | **P value¹** |
|---|---|---|---|
| Number | 5 | 5 | n/a |
| Age (years) | 8.9 ± 3.3 (5.7-13.6) | 9.2 ± 3.8 (6.2-14.7) | 0.88 |
| Leiter IQ | 82 ± 7.8 (75-92) | 79 ± 8.8 (66-87) | 0.69 |
| ADOS Score | 8.6 ± 0.9 (8-10) | 9.4 ± 1.3 (7-10) | 0.30 |
| Weight (kg) | 32 ± 14 (23-55) | 40 ± 23 (24-80) | 0.53 |
| Weight Percentile | 64 ± 16 (42-84) | 78 ± 30 (25-98) | 0.40 |
| Height (cm) | 136 ± 23 (118-174) | 137 ± 28 (113-180) | 0.92 |
| BSA* (m²) | 1.09 ± 0.32 (0.87-1.63) | 1.21 ± 0.46 (0.87-1.99) | 0.64 |
| Body Mass Index (kg/m²) | 16.8 ± 1.1 (15.5-18.1) | 19.9 ± 3.1 (16.2-24.7) | 0.07 |
| Head Circumference (cm) | 54.3 ± 2.8 (51.5-57.5) | 54.5 ± 2.3 (51.5-57) | 0.90 |
| HC Percentile | 75 ± 30 (35-99) | 75 ± 27 (42-97) | 0.97 |
| Age at ASD diagnosis (yrs.) | 3.2 ± 0.5 (2.5-3.75) | 2.7 ± 0.3 (2.5-3.0) | 0.10 |
| Paternal age at birth (yrs.) | 37 ± 3.2 (35-41) | 43 ± 12 (33-64) | 0.62 |
| Maternal age at birth (yrs.) | 35 ± 2.8 (32-38) | 41 ± 6 (33-47) | 0.053 |
| Sibling with ASD | 0 | 1 | 0.99 |
| History of GI issues-current | 0 | 1 | 0.99 |
| Maintains a gluten-free diet | 0 | 1 | 0.99 |
| IVF conception | 1 | 0 | 0.99 |
| C-section delivery | 1 | 1 | 0.99 |
| History of premature birth | 0 | 1 | 0.99 |
| History of epilepsy²-current | 0 | 0 | 0.99 |
| History of developmental regression(s) | 3 | 2 | 0.99 |
| History of asthma-current | 0 | 0 | 0.99 |
| ASD symptom improvement with fever | 2 | 1 | 0.99 |

| | | | |
|---|---|---|---|
| *Mosteller method. **Abbreviations:** BSA: body surface area; HC: head circumference; GI: gastrointestinal; IVF: in vitro fertilization; ASD: autism spectrum disorder. ¹Student's *t*-test for continuous data; Fisher's exact test for categorical data. ²Patients taking prescription drugs were excluded from the study. This included anticonvulsant medications. | | | |

### Example 2: Safety/Efficacy Analysis for Suramin treatment in Autism

Each child was used as his own control to examine before and after treatment effects from **Example 1** in a paired t-test design for the analysis of the ADOS, EOWPVT, ABC, ATEC, RBQ (**Figs. 4-11** show individual analyses), and blood and urine safety data (**Figs. 2** and **3** show individual analyses). Paired, non-parametric analysis was by Wilcoxon signed-rank sum test. Categorical data, such as the presence or absence of adverse events or historical symptoms was analyzed by Fisher's exact test. Two-way ANOVA (treatment x time), with Sidak post hoc correction, was used to analyze the 6-week summaries captured by the ADOS, CGI, and additional blood and urine safety analysis. Cohen's d-calculated as the mean difference of the paired, within-subject scores before and after treatment, divided by the standard deviation of the differences-was used as an estimate of effect size.

Extensive monitoring revealed no serious toxicities (CTCAE grades 3-5). Neurologic examinations showed there was no peripheral neuropathy (**Table 2**). Analysis of free cortisol, hemoglobin, white blood cell count (WBC), platelets, liver transaminases, creatinine, and urine protein showed no differences in children who received suramin and placebo **(****Figs. 2** and **3****).** Five children who received suramin developed a self-limited, evanescent, asymptomatic, fine macular, patchy, morbilliform rash over 1%-20% of their body (**Fig. 3D****/E**). This peaked 1 day after the infusion and disappeared spontaneously in 2-4 days. The rash was not raised and did not itch. It was not urticarial. The children did not appear to notice it. Any residual rash was covered by clothing and not visible on exposed skin at the 2-day evaluation. Parents were instructed not to discuss it with the neuropsychology team to decrease the chance of examiner bias. Video camera records of the ADOS testing confirmed the absence of any visible rash. The rash was a known risk of suramin treatment that was described in the informed consent documents. An independent data safety monitoring board (DSMB) reviewed this information, as well as the clinical safety and toxicity data and IRB communications from the study and found no safety concerns.

A single, 20 mg/kg intravenous dose of suramin was associated with improved scores for language, social interaction, and decreasing restricted or repetitive behaviors measured by ADOS, ABC, ATEC, and CGI scores **(Table 3).** None of these improvements occurred in the 5 children who received placebo.

The primary outcome measures were ADOS-2 and expressive one-word picture vocabulary (EOWPVT) scores **(Table 3).** Parents reported that after suramin treatment, the rate of language, social, behavioral, and developmental changes continued to increase for 3 weeks, and then gradually decreased toward baseline over the next 3 weeks. ADOS-2 comparison scores improved by an average of -1.6 ± 0.55 points (mean ± SD; n = 5; 95% CI = -2.3 to -0.9; Cohen's d = 2.9; p = 0.0028) at 6-weeks in the suramin treatment group and did not change in the saline group. To be conservative, we have calculated p values by both parametric and nonparametric methods (**Table 3**). The mean ADOS comparison score for this group was 8.6 ± 0.4 at baseline and 7.0 ± 0.3 6-weeks after suramin. Two-way ANOVA of ADOS scores of suramin and placebo groups measured at baseline and at 6-weeks were also significant (treatment x time interaction F(1,8) = 12.0; p = 0.0085; **Fig. 4A**). ADOS scores were not changed in the saline treated group (**Table 3**). EOWPVT scores did not change (**Table 3**).

Several secondary outcome measures also showed improvements. These included improvements in ABC, ATEC, and CGI scores (**Table 3**). The Repetitive Behavior Questionnaire (RBQ) scores did not capture a change. Four of 24 symptoms covered in the CGI were significant (**Fig. 10C**). Parents were also asked to specify the three top, most-changed behaviors as an unstructured component of the CGI at 6-weeks after the infusion. Five symptoms were named that achieved statistically significant results. These were social communication and play, speech and language, calm and focus, stims or stereotypies, and coping skills (**Fig. 10C**).

**Table 2: Summary of Adverse or Unanticipated Events**

| **No.** | **Events** | **Suramin (N=5)** | **CTCAE¹ Grade** | **Placebo (N=5)** | **CTCAE¹ Grade** | **P Value²** |
|---|---|---|---|---|---|---|
| 1 | Asymptomatic rash | 5 | 1 | 0 | - | 0.0079 |
| 2 | Uncomplicated URI³ | 2 | 1 | 2 | 1 | 0.99 |
| 3 | Headache | 1 | 1 | 0 | - | 0.99 |
| 4 | Emesis x 1 | 1⁴ | 1 | 1⁵ | 1 | 0.99 |
| 5 | Hyperactivity | 2⁶ | 1 | 1 | 1 | 0.99 |
| 6 | Hypoglycemia⁷ | 1 | 2 | 1 | 2 | 0.99 |
| 7 | Leukocytosis | 0 | - | 1⁸ | 1 | 0.99 |
| 8 | Enuresis | 1⁹ | 1 | 0 | - | 0.99 |
| 9 | Peripheral neuropathy | 0 | - - | 0 | - - | 0.99 |
| | **Total:** | 13 | - | 6 | - | 0.12 |
| | **Total, excluding rash:** | 8 | - | 6 | - | 0.77 |

| | | | | | | |
|---|---|---|---|---|---|---|
| ¹CTCAE: Common terminology criteria for adverse events v4.03. Mild to moderate = Grade 1-2; Serious = Grade 3-5. ²Fisher's exact test. ³URI: upper respiratory tract infection, common cold. The study was conducted October-March. ⁴In 7-year old after pizza and slushee consumption after playing youth league basketball. ⁵In a 6-year old after a car ride. ⁶In a 5 and 14-year old intermixed with periods of calm focus in first week (the 14-year old) or first 3 weeks (the 5-year old). ⁷6-weeks after the infusion, after several days of a cold and fasting before lunch. Hypoglycemia was asymptomatic and corrected after a normal lunch. ⁸Leukocytosis (12.2k WBC) occurred on the day of the saline infusion and preceded a URI. ⁹In a 7-year old briefly for a few days while sick with a cold. None of the events required medical intervention. No serious adverse events (SAEs) occurred in this study. | | | | | | |

**Table 3: Behavioral Outcomes for Suramin or Placebo Treatment**

| **Outcome** | | | | **Suramin** | | | | | **Placebo** | | | | | |
|---|---|---|---|---|---|---|---|---|---|---|---|---|---|---|
| Instrument | Factor or behavior | Time after treatment (days) | Difference from baseline (mean ± sd) | 95% CI | d¹ | N | P² | P³ | Difference from baseline (mean ± sd) | 95% CI | d¹ | N | P² | P³ |
| **Primary Outcomes** | | | | | | | | | | | | | | |
| ADOS-2 | Comparison | 45 | -1.6 ± 0.55 | -2.3 to - 0.9 | 2.9 | 5 | 0.0028 | 0.038 | -0.4 ± 0.55 | -1.1 to +0.28 | 0.7 | 5 | 0.18 | 0.16 |
| | Raw | 45 | -4.6 ± 1.9 | -7.0 to - 2.2 | 2.4 | 5 | 0.0062 | 0.039 | -0.4 ± 1.8 | -2.7 to +1.9 | 0.22 | 5 | 0.65 | 0.58 |
| | Social | 45 | -3.2 ± 1.9 | -5.6 to - 0.8 | 1.7 | 5 | 0.020 | 0.043 | 0.0 ± 1.7 | -2.2 to +2.2 | 0 | 5 | 0.99 | 0.71 |
| | Restr/Rep | 45 | -1.4 ± 0.89 | -2.5 to - 0.29 | 1.6 | 5 | 0.025 | 0.059 | -0.4 ± 2.1 | -3.0 to +2.2 | 0.19 | 5 | 0.69 | 0.58 |
| EOWPVT | Vocabulary | 45 | -4.2 ± 8.3 | 14.5 to +6.1 | 0.51 | 5 | 0.32 | 0.50 | +2.0 ± 4.6 | -3.8 to +7.8 | 0.43 | 5 | 0.39 | 0.50 |

| **Secondary Outcomes** | | | | | | | | | | | | | | |
|---|---|---|---|---|---|---|---|---|---|---|---|---|---|---|
| ABC | Stereotypy | 7 | -3.6 ± 2.1 | -6.2 to - 1.0 | 1.7 | 5 | 0.018 | 0.043 | +0.4 ± 1.9 | -2.0 to +2.8 | 0.21 | 5 | 0.67 | 0.68 |

| **Outcome** | | | **Suramin** | | | | | | **Placebo** | | | | | |
|---|---|---|---|---|---|---|---|---|---|---|---|---|---|---|
| | Stereotypy | 45 | -4.0 ± 2.3 | -6.9 to - 1.1 | 1.7 | 5 | 0.019 | 0.042 | +1.0 ± 4.3 | -4.3 to +6.3 | 0.23 | 5 | 0.63 | 0.69 |
| ATEC | Total | 7 | -10 ± 7.7 | -20 to - 0.46 | 1.3 | 5 | 0.044 | 0.043 | +7.2 ± 14 | -10 to +25 | 0.51 | 5 | 0.32 | 0.35 |
| | Language | 7 | -2.2 ± 1.5 | -4.0 to - 0.36 | 1.4 | 5 | 0.021 | 0.059 | 0.0 ± 4.1 | -5.0 to +5.0 | 0 | 5 | 0.99 | 0.89 |
| | Sociability | 7 | -3.6 ± 2.6 | -6.8 to - 0.36 | 1.4 | 5 | 0.025 | 0.063 | -0.8 ± 2.8 | -4.3 to +2.6 | 0.29 | 5 | 0.55 | 0.58 |
| | Language | 45 | -2.0 ± 1.4 | -2.7 to - 0.49 | 1.4 | 5 | 0.034 | 0.059 | -0.2 ± 2.9 | -3.8 to +3.4 | 0.07 | 5 | 0.88 | 0.79 |
| CGI | Overall ASD | 45 | -1.8 ± 1.04 | -3.4 to - 0.15 | 1.7 | 5 | 0.05 | n/a | 0.0 ± 0.34 | -0.55 to +0.55 | 0 | 5 | 0.99 | n/a |
| | E. Language | 45 | -2.0 ± 1.04 | -3.6 to - 0.35 | 1.9 | 5 | 0.01 | n/a | 0.0 ± 0.34 | -0.55 to +0.55 | 0 | 5 | 0.99 | n/a |
| | Social Inter. | 45 | -2.0 ± 1.04 | -3.6 to - 0.35 | 1.9 | 5 | 0.01 | n/a | 0.0 ± 0.34 | -0.55 to +0.55 | 0 | 5 | 0.99 | n/a |
| RBQ | Total | 45 | -3.2 ± 5.8 | 10.4 to +4.0 | 0.55 | 5 | 0.28 | 0.22 | -0.8 ± 3.3 | -4.9 to 3.3 | 0.24 | 5 | 0.62 | 0.47 |

| **Outcome** | **Suramin** | **Placebo** | | | | | | | | | | | | |
|---|---|---|---|---|---|---|---|---|---|---|---|---|---|---|
| ¹Positive Cohen's d reflects improvement by convention. Cohen's d is likely an overestimate of the actual treatment effect based on the large mean differences and small standard deviations found before and after treatment in this small study. ²P value using parametric paired *t*-test analysis. ³P value using the non-parametric paired Wilcoxon signed-rank sum analysis. | | | | | | | | | | | | | | |
| **Abbreviations.** ADOS-2: autism diagnostic observation schedule, 2^{nd} edition. EOWPVT: expressive one-word picture vocabulary test. ABC: aberrant behavior checklist. ATEC: autism treatment evaluation checklist. CGI: clinical global impression survey. RBQ: repetitive behavior questionnaire. Restr/Rep: restricted or repetitive behaviors. Overall ASD Sx: overall ASD symptoms. E. Language: expressive language. Social Inter.: social interaction. **Analysis.** ADOS, EOWPVT, ABC, ATEC, and RBQ scores were analyzed by paired analysis before and after treatment using each subject as their own control. CGI was analyzed by 2-way ANOVA (symptom x time before and after treatment) with post hoc correction. Non-parametric p values were not calculated (n/a). **Interpretation.** ADOS, ABC, ATEC, CGI, and RBQ are severity scores; negative differences from baseline reflect decreased severity, i.e., improvement. EOWPVT is a performance score; negative differences reflect a decrease. | | | | | | | | | | | | | | |

### Example 3: Suramin pharmacokinetics in pediatric patients

Suramin concentrations were measured by high performance liquid chromatography and tandem mass spectrometry (LC-MS/MS) on plasma samples collected before the infusion, at 1 hour, 2 days, and 45 days post-infusion for the study described in **Example 1.**

Heparinized plasma, 90 µl was prepared for LC-MS/MS analysis. Ten (10) µl of 50 µM stock of trypan blue was added to achieve an internal standard concentration of 5 µM. This was incubated at room temperature for 10 min to permit metabolite interaction with binding proteins, then extracted with 4 volumes (400 µl) of pre-chilled methanol-acetonitrile (50:50) to produce a final concentration of 40:40:20 (methanol:acetonitrile:H2O), and precipitated on ice for 10 minutes. The samples were deproteinated and macromolecules removed by precipitation on crushed ice for 10 min.
The mixture was centrifuged at 16,000g for 10 min at 4°C and the supernatant was transferred to a new tube and kept at -80°C for further LC-MS/MS analysis.

Suramin was detected in the prepared samples on an AB SCIEX QTRAP 5500 triple quadrupole mass spectrometer equipped with a Turbo V electrospray ionization (ESI) source, Shimadzu LC-20A UHPLC system, and a PAL CTC autosampler. Ten microliters of extract were injected onto a Kinetix F5 column (100 × 2.1 mm, 2.6 µM; Phenomenex, CA) held at 30°C for chromatographic separation. The mobile phase A was water with 20 mM ammonium acetate (NH4OAC) (pH 7) and mobile phase B was methanol with 20 mM NH4OAC (pH 7). Elution was performed using the following gradient: 0-1.5 min-0% B, 1.6-3 min-15% B, 3.1-7 min-60% B, 7.1-13 min-100% B, 14 min-0% B, 18 min-0% B, 18.1 minute-end. The flow rate was 400 µl/min. All the samples were kept at 4°C during analysis. Suramin and trypan blue were detected using MRM scanning mode with the dwell time of 180 ms. MRM transitions for the doubly-charged form of suramin were 647.0 m/z for the (Q1) precursor and 382.0 m/z for the (Q3) product. MRM transitions for trypan blue were 435.2 (Q1) and 185.0 (Q3). Absolute concentrations of suramin were determined using a standard curve prepared in plasma to account for matrix effects, and the peak area ratio of suramin to the internal standard trypan blue. The declustering potential (DP), collision energy (CE), entrance potential (EP) and collision exit potential (CXP) were -104, -9.5, -32 and -16.9, and -144.58, -7, -57.8 and -20.94, for suramin and trypan blue, respectively. The ESI source parameters were set as follows: source temperature 500 °C; curtain gas 30; ion source gas 1, 35; ion source gas 2 35; spray voltage -4500 V. Analyst v1.6 was used for data acquisition and analysis.

The small number of PK samples per subject prevented a standard, non-compartmental analysis in individual subjects. The suramin drug concentrations were analyzed using a population PK approach with post-hoc empiric Bayesian estimate of PK parameters in individual subjects. The PK data were fit to a two compartment model using the computer program NONMEM (ICON, Dublin, Ireland). PK parameters were scaled allometrically with volume terms scaled to linear body weight (kg1.0) and clearance terms scaled to weight (kg0.75). Scaled adult suramin parameters of compartmental volumes of distribution and clearance were used as initial parameter estimates and between subject variability only estimated for clearance (CL) and the peripheral volume of distribution (Vd).

A two-compartment pharmacokinetic model permitted accurate forecasting of plasma drug levels from 1 hour to 6-weeks after the infusion. Pharmacokinetic analysis showed that at 1 hour after intravenous infusion of 20 mg/kg (558 ± 41 mg/m2; mean ± sd; see **Table 4**), the suramin concentration was 104 ± 11.6 µM (**Fig. 12A**). The alpha (distribution) phase half-life was 7.4 ± 0.55 hours. The suramin levels rapidly fell below 100 µM and into the target range before Day 2 in all subjects, with an average plasma level of suramin of 12.0 ± 1.5 µM on Day 2 **(****Fig. 12B****).** Target concentrations of 1.5-15 µM were maintained between 2 days and 5-6 weeks following the dose **(****Fig. 11****).** The steady-state volume of distribution was 0.83 ± 0.014 L/kg (22.7 ± 2.6 L/m2). The clearance was 1.95 ± 0.21 mL/hr/kg (0.056 ± 0.011 L/hr/m2). The terminal elimination phase half-life (t1/2) was 14.7 ± 1.4 days **(Fig. 12CD).** The low variability in pharmacokinetic parameters was consistent with the low variability in the day 2 concentrations (CV = 12%).

These data are the first in the published literature on the pharmacokinetics of suramin in a pediatric population.

**Table 4: Single-dose Suramin Pharmacokinetics**

| **Pair Block** | **ID** | **Age (yrs.)** | **Height (m)** | **Weight (kg)** | **BSA* (m²)** | **20 mg/kg Dose (mg)** | **Dose (mg/m²)** | **1-Hour Plasm a Conc (µM)** | **2-Day Plasma Conc** (µM) | **45-Day Plasma Conc** (µM) | **Plasma Half-Life (days)** |
|---|---|---|---|---|---|---|---|---|---|---|---|
| 1 | 001 | 11 | 1.395 | 34.4 | 1.15 | 680 | 591 | 101.2 | 13.2 | 0.96 | 12.6 |
| 2 | 007 | 5 | 1.189 | 22.9 | 0.87 | 460 | 529 | 87.9 | 11.9 | 1.67 | 14.7 |
| 3 | 014 | 14 | 1.74 | 54.7 | 1.63 | 1000 | 613 | 110.9 | 10.6 | 1.04 | 14.9 |
| 4 | 012 | 6 | 1.18 | 23.1 | 0.87 | 460 | 529 | 118.6 | 13.8 | 2.28 | 16.5 |
| 5 | 005 | 7 | 1.271 | 25.1 | 0.95 | 500 | 526 | 101.8 | 10.6 | 1.76 | 15.0 |
| | | | | | | **Mean:** | 558 | 104.1 | 12.0 | 1.54 | 14.7 |
| | | | | | | **sd:** | 41 | 11.6 | 1.5 | 0.6 | 1.4 |

| | | | | | | | | | | | |
|---|---|---|---|---|---|---|---|---|---|---|---|
| *Mosteller method. | | | | | | | | | | | |

### Example 4: Metabolomic Analysis of Suramin Biological Effects in Pediatric Patients

Targeted, broad-spectrum, plasma metabolomic analysis of 610 metabolites from 63 biochemical pathways was performed by high performance liquid chromatography and tandem mass spectrometry (LC-MS/MS) on the samples collected from patients in **Example 1.** Venous blood was collected between the hours of 8 am and 5 pm, at least 3 hours after the last meal, into lithium-heparin vacutainer tubes (BD #367884). Plasma was separated by centrifugation at 900g x 10 minutes at room temperature within one hour of collection. The resulting fresh lithium-heparin plasma was transferred to labeled 1.2 ml or 2.0 ml externally threaded cryotubes with a minimum headspace air gap for storage at -80°C for analysis. Samples were analyzed on an AB SCIEX QTRAP 5500 triple quadrupole mass spectrometer equipped with a Turbo V electrospray ionization (ESI) source, Shimadzu LC-20A UHPLC system, and a PAL CTC autosampler. Typically, 90 µl of plasma was thawed on ice and transferred to a 1.7 ml Eppendorf tube. Five (5.0) µl of a cocktail containing 25-35 commercial stable isotope internal standards, and 5.0 µl of 57 stable isotope internal standards that were custom-synthesized in E. coli NCM3722, Caenorhabditis elegans N2, and Komagataella phaffii (ATCC 76273; formerly known as Pichia pastoris) by metabolic labeling with 13C-glucose and 13C-bicarbonate, were added, mixed, and incubated for 10 min at 20°C to permit small molecules and vitamins in the internal standards to associate with plasma binding proteins. Macromolecules (protein, DNA, RNA, glycans, etc.) were precipitated by extraction with 4 volumes (400 µl) of cold (-20°C), acetonitrile:methanol (50:50) (LCMS grade, Cat# LC015-2.5 and GC230-4, Burdick & Jackson, Honeywell), vortexed vigorously, and incubated on crushed ice for 10 min, then removed by centrifugation at 16,000g x 10 min at 4°C. The supernatants containing the extracted metabolites and internal standards in the resulting 40:40:20 solvent mix of acetonitrile:methanol:water were transferred to labeled cryotubes and stored at -80°C for LCMS/MS analysis.

LC-MS/MS analysis was performed by scheduled multiple reaction monitoring (sMRM) under Analyst v1.6.2 software control in both negative and positive mode with rapid polarity switching (50 ms). Nitrogen was used for curtain gas (set to 30), collision gas (set to high), ion source gas 1 and 2 (set to 35). The source temperature was 500°C. Spray voltage was set to -4500 V in negative mode and 5500 V in positive mode. The values for Q1 and Q3 mass-to-charge ratios (m/z), declustering potential (DP), entrance potential (EP), collision energy (CE), and collision cell exit potential (CXP) were determined and optimized for each MRM for each metabolite. Ten microliters of extract was injected by PAL CTC autosampler via a 10 µl stainless steel loop into a 250 mm × 2.0 mm, 4µm polymer based NH2 HPLC column (Asahipak NH2P-40 2E, Showa Denko America, Inc., NY) held at 25°C for chromatographic separation. The mobile phase was solvent A: 95% water with 20 mM (NH4)2CO3 (Sigma, Fluka Cat# 74415-250G-F), 5% acetonitrile, and 38 mM NH4OH (Sigma, Fluka Cat# 17837-100ML), final pH 9.75; solvent B: 100% acetonitrile. Separation was achieved using the following gradient: 0-3.5 min: 95%B, 3.6-8 min: 85% B, 8.1-13 min: 75% B, 13.5-35 min: 0% B, 36-46 min: 95% B, 46.1 min: end. The flow rate was 200 µl/min. Pump pressures ranged from 920-2600 psi over the course of the gradient. All the samples were kept at 4°C during analysis. The chromatographic peaks were identified using MultiQuant (v3.0, Sciex), confirmed by manual inspection, and the peak areas integrated.

Metabolomic data were log-transformed, scaled by control standard deviations, and analyzed by multivariate partial least squares discriminant analysis (PLSDA), with pairwise comparisons and post hoc correction for multiple hypothesis testing using Fisher's least significant difference method in MetaboAnalyst36, or the false discovery rate (FDR) method of Benjamini and Hochberg. Metabolites with variable importance in projection (VIP) scores determined by PLSDA that were greater than 1.5 were considered significant. Spearman non-parametric correlation methods were implemented in Stata (Stata/SE12.1, StataCorp, College Station, TX), Prism (Prism 6, GraphPad Software, La Jolla, CA), or R. Significant metabolites were grouped into pathways and their VIP scores summed to determine the rank-ordered significance of each biochemical pathway.

The small number of subjects in this trial precluded conventional treatment group analysis because of high false discovery rates associated with measuring 610 metabolites in groups with just 5 subjects. However, by using each child as their own control in a paired analysis of pre-infusion and post-infusion results, the pharmacometabolomic effects of suramin could be characterized (**Fig. 13**). The rank order of the top 35 of 48 significant metabolites 6-weeks after suramin treatment is illustrated in **Fig. 13A.** The most significantly altered metabolites were then organized according to biochemical pathways (**Table 5**). The majority of the pathways altered by suramin were known features of the cell danger response.

Metabolomic studies confirmed the importance of the cell danger response (CDR) and purinergic signaling; as the metabolic effects of suramin resulted in a decrease of the cell danger response and restored more normal metabolism. Purine metabolism was the single most changed pathway. Suramin increased healthy purines such as AICAR, which is an activator of the master metabolic regulator AMP kinase. 1-Methyl-adenine (1-MA) was also increased. 1-MA is derived from 1-methyl-adenosine, a recently recognized marker of new protein synthesis and cell growth. Suramin decreased other purines in the plasma such as cAMP and dGDP (**Fig. 13**, **Tables 6-8**).

Although no behavioral outcomes were significant at 2 days after infusion, we found that 28 biochemical pathways were changed by suramin 2-days after the infusion **(Table** 6). Twenty-two of these (79%) remained changed at the 6-week time point **(Table** 5). The rank order of metabolites most changed at day 2, and their associated metabolic pathway is illustrated in **Fig. 14****.** The full list of 61 metabolites on day 2 and 48 metabolites at 6-weeks that were significantly changed by suramin appears in **Tables 7-8.**

**Table 5: Suramin pharmacometabolomics: biochemical pathways changed at 6-weeks**

| **No.** | **Pathway Name** | **Measured Metabolites in the Pathway (N)** | **Expected Pathway Proportion (P = N/429)** | **Expected Hits in Sample of 48 (P * 48)** | **Observed Hits in the Top 48 Metabolites** | **Fold Enrichment (Obs/Exp)** | **Impact (Sum VIP Score)** | **Fraction of Impact (VIP Score) Explained (% of 94.6)** | **Increased** | **Decreased** |
|---|---|---|---|---|---|---|---|---|---|---|
| 1 | Purine Metabolism | 26 | 0.061 | 2.9 | 5 | 1.7 | 10.2 | 11% | 3 | 2 |
| 2 | SAM, SAH, Methionine, Cysteine, Glutathione | 15 | 0.035 | 1.7 | 5 | 3.0 | 9.5 | 10% | 5 | 0 |
| 3 | Microbiome Metabolism | 18 | 0.042 | 2.0 | 4 | 2.0 | 8.4 | 9% | 4 | 0 |
| 4 | Branch Chain Amino Acid Metabolism | 12 | 0.028 | 1.3 | 4 | 3.0 | 7.4 | 8% | 4 | 0 |
| 5 | Bile Acid Metabolism | 6 | 0.014 | 0.7 | 3 | 4.5 | 5.7 | 6% | 3 | 0 |
| 6 | Fatty Acid Oxidation and Synthesis | 37 | 0.086 | 4.1 | 3 | 0.7 | 5.0 | 5% | 0 | 3 |
| 7 | Amino Acid Metabolism (not otherwise covered) | 4 | 0.009 | 0.4 | 2 | 4.5 | 4.3 | 5% | 2 | 0 |
| 8 | Krebs Cycle | 9 | 0.021 | 1.0 | 2 | 2.0 | 4.3 | 5% | 2 | 0 |
| 9 | Pyrimidine Metabolism | 9 | 0.021 | 1.0 | 2 | 2.0 | 4.2 | 4% | 2 | 0 |
| 10 | Sphingomyelin Metabolism | 36 | 0.084 | 4.0 | 2 | 0.5 | 4.1 | 4% | 2 | 0 |
| 11 | 1-Carbon, Folate, Formate, Glycine, Serine Metabolism | 5 | 0.012 | 0.6 | 2 | 3.6 | 4.0 | 4% | 2 | 0 |
| 12 | GABA, Glutamate, Arginine, Omithine, Proline | 6 | 0.014 | 0.7 | 2 | 3.0 | 3.9 | 4% | 2 | 0 |
| 13 | Tyrosine and Phenylalanine Metabolism | 3 | 0.007 | 0.3 | 2 | 6.0 | 3.7 | 4% | 2 | 0 |
| 14 | Cholesterol, Cortisol, Non-Gonadal Steroid Metabolism | 16 | 0.037 | 1.8 | 2 | 1.1 | 3.5 | 4% | 2 | 0 |
| 15 | Gamma-Glutamyl and other Dipeptides | 2 | 0.005 | 0.2 | 1 | 4.5 | 2.4 | 2% | 1 | 0 |
| 16 | Histidine, Histamine, Camosine Metabolism | 4 | 0.009 | 0.4 | 1 | 2.2 | 2.3 | 2% | 1 | 0 |
| 17 | Nitric Oxide, Superoxide, Peroxide Metabolism | 2 | 0.005 | 0.2 | 1 | 4.5 | 2.2 | 2% | 1 | 0 |
| 18 | Tryptophan, Kynurenine, Serotonin, Melatonin | 6 | 0.014 | 0.7 | 1 | 1.5 | 2.1 | 2% | 1 | 0 |
| 19 | Glycolysis and Gluconeogenesis Metabolism | 7 | 0.016 | 0.8 | 1 | 1.3 | 2.1 | 2% | 1 | 0 |
| 20 | Vitamin C (Ascorbate) Metabolism | 2 | 0.005 | 0.2 | 1 | 4.5 | 2.0 | 2% | 1 | 0 |
| 21 | Amino-Sugar, Galactose, & Non-Glucose Metabolism | 5 | 0.012 | 0.6 | 1 | 1.8 | 1.9 | 2% | 1 | 0 |
| 22 | Phospholipid Metabolism | 73 | 0.170 | 8.2 | 1 | 0.1 | 1.6 | 2% | 0 | 1 |
| | | | | | | | | **Subtotal:** | 42 | 6 |
| | | | | | | | | **Total:** | 48 | |

**Table 6: Suramin pharmacometabolomics. Pathways changed at 2-days**

| **No.** | **Pathway Name** | **Measured Metabolites in the Pathway (N)** | **Expected Pathway Proportion (P = N/431)** | **Expected Hits in Sample of 61 (P * 61)** | **Observed Hits in the Top 61 Metabolites** | **Fold Enrichment (Obs/Exp)** | **Impact (Sum VIP Score)** | **Fraction of Impact (VIP Score) Explained (% of 119.7)** | **Increased** | **Decreased** |
|---|---|---|---|---|---|---|---|---|---|---|
| **No.** | **Pathway Name** | **Measured Metabolites in the Pathway (N)** | **Expected Pathway Proportion (P = N/431)** | **Expected Hits in Sample of 61 (P * 61)** | **Observed Hits in the Top 61 Metabolites** | **Fold Enrichment (Obs/Exp)** | **Impact (Sum VIP Score)** | **Fraction of Impact (VIP Score) Explained (% of 119.7)** | **Increased** | **Decreased** |
| 1 | Purine Metabolism | 26 | 0.060 | 3.7 | 9 | 2.4 | 17.6 | 15% | 4 | 5 |
| 2 | Bile Salt Metabolism | 6 | 0.014 | 0.8 | 4 | 4.7 | 11.9 | 10% | 4 | 0 |
| 3 | Microbiome Metabolism | 18 | 0.042 | 2.5 | 4 | 1.6 | 9.3 | 8% | 4 | 0 |
| 4 | Branch Chain Amino Acid Metabolism | 12 | 0.028 | 1.7 | 4 | 2.4 | 7.3 | 6% | 4 | 0 |
| 5 | Eicosanoid and Resolvin Metabolism | 13 | 0.030 | 1.8 | 4 | 2.2 | 7.1 | 6% | 0 | 4 |
| 6 | Phospholipid Metabolism | 74 | 0.172 | 10.5 | 3 | 0.3 | 5.7 | 5% | 0 | 3 |
| 7 | SAM, SAH, Methionine, Cysteine, Glutathione | 15 | 0.035 | 2.1 | 3 | 1.4 | 5.6 | 5% | 2 | 1 |
| 8 | GABA, Glutamate, Arginine, Ornithine | 6 | 0.014 | 0.8 | 3 | 3.5 | 4.7 | 4% | 3 | 0 |
| 9 | Pyrimidine Metabolism | 9 | 0.021 | 1.3 | 2 | 1.6 | 4.3 | 4 | 1 | 1 |
| 10 | Glycolysis and Gluconeogenesis Metabolism | 7 | 0.016 | 1.0 | 2 | 2.0 | 4.3 | 4% | 2 | 0 |
| 11 | Gamma-Glutamyl and other Dipeptides | 2 | 0.005 | 0.3 | 2 | 7.1 | 3.8 | 3% | 2 0 | 0 2 |
| 12 | Sphingomyelin Metabolism | 36 | 0.084 | 5.1 | 2 | 0.4 | 3.6 | 3% | 0 | 2 |
| 13 | Bioamines and Neurotransmitter Metabolism | 9 | 0.021 | 1.3 | 2 | 1.6 | 3.3 | 3% | 0 | 2 |
| 14 | Krebs Cycle | 9 | 0.021 | 1.3 | 2 | 1.6 | 3.3 | 3% | 2 | 0 |
| 15 | Vitamin D (Calciferol) Metabolism | 3 | 0.007 | 0.4 | 1 | 2.4 | 3.1 | 3% | 0 | 1 |
| 16 | Cardiolipin Metabolism | 7 | 0.016 | 1.0 | 2 | 2.0 | 3.1 | 3% | 2 | 0 |
| 17 | Glycosphingolipi d Metabolism | 12 | 0.028 | 1.7 | 1 | 0.6 | 2.1 | 2% | 1 | 0 |
| 18 | Taurine, Hypotaurine Metabolism | 2 | 0.005 | 0.3 | 1 | 3.5 | 2.0 | 2% | 0 | 1 |
| 19 | Nitric Oxide, Superoxide, Peroxide | 2 | 0.005 | 0.3 | 1 | 3.5 | 1.9 | 2% | 0 | 1 |
| 20 | Histidine, Histamine, Carnosine Metabolism | 4 | 0.009 | 0.6 | 1 | 1.8 | 1.8 | 2% | 1 | 0 |
| 21 | Tyrosine and Phenylalanine Metabolism | 3 | 0.007 | 0.4 | 1 | 2.4 | 1.8 | 2% | 1 | 0 |
| 22 | Fatty Acid Oxidation and Synthesis | 37 | 0.086 | 5.2 | 1 | 0.2 | 1.8 | 2% | 0 | 1 |
| 23 | Cholesterol, Cortisol, Non-Gonadal Steroid | 16 | 0.037 | 2.3 | 1 | 0.4 | 1.8 | 2% | 1 | 0 |
| 24 | Amino Acid Metabolism | 4 | 0.009 | 0.6 | 1 | 1.8 | 1.8 | 1% | 1 | 0 |
| 25 | Endocannabinoi d Metabolism | 4 | 0.009 | 0.6 | 1 | 1.8 | 1.7 | 1% | 0 | 1 |
| 26 | Amino-Sugar, Galactose, & Non-Glucose | 5 | 0.012 | 0.7 | 1 | 1.4 | 1.6 | 1% | 1 | 0 |
| 27 | Tryptophan, Kynurenine, Serotonin | 6 | 0.014 | 0.8 | 1 | 1.2 | 1.6 | 1% | 1 | 0 |
| 28 | Ceramide Metabolism | 34 | 0.079 | 4.8 | 1 | 0.2 | 1.5 | 1% | 1 | 0 |
| | | | | | | | | **Subtotals** | 38 | 23 |
| | | | | | | | | **Totals:** | 61 | |

**Table 7: Suramin pharmacometabolomics. Metabolites changed at 2-days**

| **No.** | **Metabolite** | **Pathway Name** | **VIP Score** | **Z Score** | **Z Abs** | **Linear Ratio** |
|---|---|---|---|---|---|---|
| 1 | Chenodeoxyglycocholic acid | Bile Salt Metabolism | 3.171 | 1.610 | 1.610 | 2.787 |
| 2 | 1,25-Dihydroxyvitamin D3 | Vitamin D (Calciferol) Metabolism | 3.134 | -1.447 | 1.447 | 0.273 |
| 3 | Glycocholic acid | Bile Salt Metabolism | 3.090 | 2.020 | 2.020 | 2.344 |
| 4 | Taurodeoxycholic acid Pool | Bile Salt Metabolism | 3.048 | 1.326 | 1.326 | 2.614 |
| 5 | 2-Keto-L-gluconate | Microbiome Metabolism | 2.994 | 2.586 | 2.586 | 1.264 |
| 6 | Taurocholic acid | Bile Salt Metabolism | 2.615 | 1.102 | 1.102 | 2.183 |
| 7 | 2,3-Diphosphoglyceric acid | Glycolysis and Gluconeogenesis Metabolism | 2.600 | 0.990 | 0.990 | 1.198 |
| 8 | Cytosine | Pyrimidine Metabolism | 2.556 | 2.055 | 2.055 | 1.689 |
| 9 | p-Hydroxyphenylacetic acid | Microbiome Metabolism | 2.546 | 1.464 | 1.464 | 1.192 |
| 10 | 11(R)-HETE | Eicosanoid and Resolvin Metabolism | 2.400 | -0.875 | 0.875 | 0.748 |
| 11 | Hypoxanthine | Purine Metabolism | 2.267 | -1.000 | 1.000 | 0.745 |
| 12 | Deoxyguanosine diphosphate | Purine Metabolism | 2.264 | -1.276 | 1.276 | 0.889 |
| 13 | Glycylproline | Gamma-Glutamyl and other Dipeptides | 2.205 | 1.212 | 1.212 | 1.773 |
| 14 | Allantoin | Purine Metabolism | 2.195 | 0.926 | 0.926 | 1.663 |
| 15 | L-Isoleucine | Branch Chain Amino Acid Metabolism | 2.136 | 0.815 | 0.815 | 1.094 |
| 16 | GC(18:1/22:0) | Glycosphingolipid Metabolism | 2.123 | 1.057 | 1.057 | 1.399 |
| 17 | Cysteamine | SAM, SAH, Methionine, Cysteine, Glutathione Metabolism | 2.075 | 1.398 | 1.398 | 1.107 |
| 18 | LysoPC(16:0) | Phospholipid Metabolism | 2.067 | -0.908 | 0.908 | 0.777 |
| 19 | Taurine | Taurine, Hypotaurine Metabolism | 2.042 | -0.942 | 0.942 | 0.786 |
| 20 | 1-Methyladenine | Purine Metabolism | 2.033 | 1.337 | 1.337 | 1.631 |
| 21 | SM(d18:1/20:1) | Sphingomyelin Metabolism | 2.033 | -1.250 | 1.250 | 0.745 |
| 22 | PA(16:0/16:1) | Phospholipid Metabolism | 1.998 | -0.813 | 0.813 | 0.793 |
| 23 | Cyclic adenosine monophosphate | Purine Metabolism | 1.949 | -0.681 | 0.681 | 0.855 |
| 24 | Azelaic acid | Nitric Oxide, Superoxide, Peroxide Metabolism | 1.929 | -2.024 | 2.024 | 0.914 |
| 25 | Shikimate-3 -phosphate | Microbiome Metabolism | 1.886 | 1.033 | 1.033 | 1.047 |
| 26 | Indoxyl sulfate | Microbiome Metabolism | 1.858 | 0.702 | 0.702 | 1.280 |
| 27 | 1 -Methylhistidine | Histidine, Histamine, Carnosine Metabolism | 1.848 | 0.899 | 0.899 | 1.145 |
| 28 | Purine | Purine Metabolism | 1.847 | 1.137 | 1.137 | 1.203 |
| 29 | L-Phenylalanine | Tyrosine and Phenylalanine Metabolism | 1.839 | 0.957 | 0.957 | 1.164 |
| 30 | Malonic acid | Fatty Acid Oxidation and Synthesis | 1.833 | -0.825 | 0.825 | 0.904 |
| 31 | Methionine sulfoxide | SAM, SAH, Methionine, Cysteine, Glutathione Metabolism | 1.817 | 1.738 | 1.738 | 1.331 |
| 32 | L-Valine | Branch Chain Amino Acid Metabolism | 1.808 | 0.749 | 0.749 | 1.165 |
| 33 | 24,25-Epoxycholesterol | Cholesterol, Cortisol, Non-Gonadal Steroid Metabolism | 1.807 | 1.014 | 1.014 | 1.362 |
| 34 | Orotic acid | Pyrimidine Metabolism | 1.787 | -0.612 | 0.612 | 0.670 |
| 35 | AICAR | Purine Metabolism | 1.787 | 1.310 | 1.310 | 1.309 |
| 36 | Isovalerylgl ycine | Branch Chain Amino Acid Metabolism | 1.783 | 0.852 | 0.852 | 1.951 |
| 37 | Alanine | Amino Acid Metabolism (not otherwise covered) | 1.776 | 1.066 | 1.066 | 1.193 |
| 38 | Xanthosine | Purine Metabolism | 1.764 | -1.316 | 1.316 | 0.821 |
| 39 | Anandamide | Endocannabinoid Metabolism | 1.713 | -0.709 | 0.709 | 0.684 |
| 40 | Citramalic acid | Krebs Cycle | 1.704 | 1.229 | 1.229 | 1.121 |
| 41 | Cysteine-S-sulfate | SAM, SAH, Methionine, Cysteine, Glutathione Metabolism | 1.682 | -0.644 | 0.644 | 0.869 |
| 42 | PG(16:0/16:0) | Phospholipid Metabolism | 1.664 | -0.667 | 0.667 | 0.549 |
| 43 | Dopamine | Bioamines and Neurotransmitter Metabolism | 1.653 | -0.642 | 0.642 | 0.877 |
| 44 | Glycerol 3-phosphate | Glycolysis and Gluconeogenesis Metabolism | 1.651 | 1.151 | 1.151 | 1.187 |
| 45 | 5-HETE | Eicosanoid and Resolvin Metabolism | 1.646 | -0.671 | 0.671 | 0.866 |
| 46 | Myoinositol | Amino-Sugar, Galactose, & Non-Glucose Metabolism | 1.645 | 0.785 | 0.785 | 1.286 |
| 47 | L-Glutamic acid | Bioamines and Neurotransmitter Metabolism | 1.641 | -0.619 | 0.619 | 0.797 |
| 48 | Gamma-Aminobutyric acid | GABA, Glutamate, Arginine, Ornithine, Proline Metabolism | 1.626 | 1.101 | 1.101 | 1.068 |
| 49 | L- Kynurenine | Tryptophan, Kynurenine, Serotonin, Melatonin Metabolism | 1.617 | 0.625 | 0.625 | 1.099 |
| 50 | Citric acid | Krebs Cycle | 1.590 | 0.759 | 0.759 | 1.142 |
| 51 | SM(d18:1/20:0) | Sphingomyelin Metabolism | 1.576 | -0.770 | 0.770 | 0.712 |
| 52 | Gamma-glutamyl-Alanine | Gamma-Glutamyl and | 1.575 | 0.896 | 0.896 | 1.294 |
| | | other Dipeptides | | | | |
| 53 | Tiglylglycine | Branch Chain Amino Acid Metabolism | 1.562 | 0.657 | 0.657 | 1.141 |
| 54 | L-Proline | GABA, Glutamate, Arginine, Ornithine, Proline Metabolism | 1.548 | 0.603 | 0.603 | 1.155 |
| 55 | CL(18:2/18:2/18:2/18:2) | Cardiolipin Metabolism | 1.538 | 0.506 | 0.506 | 1.181 |
| 56 | CL(18:2/18:2/18:2/18: 1) | Cardiolipin Metabolism | 1.535 | 0.474 | 0.474 | 1.102 |
| 57 | 11,12-Epoxyeicosatrienoic acid | Eicosanoid and Resolvin Metabolism | 1.535 | -0.634 | 0.634 | 0.828 |
| 58 | Ceramide(d18:1/18:2) | Ceramide Metabolism | 1.521 | 0.530 | 0.530 | 1.337 |
| 59 | Guanosine | Purine Metabolism | 1.519 | -0.766 | 0.766 | 0.702 |
| 60 | Prostaglandin J2 | Eicosanoid and Resolvin Metabolism | 1.509 | -0.601 | 0.601 | 0.649 |
| 61 | N-Acetylglutamic acid | GABA, Glutamate, Arginine, Ornithine, Proline Metabolism | 1.505 | 0.613 | 0.613 | 1.120 |

**Table 8: Suramin pharmacometabolomics. Metabolites changed at 6-weeks**

| **No.** | **Metabolite** | **Pathway Name** | **VIP Score** | **Z Score** | **Z Abs** | **Linear Ratio** |
|---|---|---|---|---|---|---|
| 1 | 2-Keto-L-gluconate | Microbiome Metabolism | 2.686 | 2.365 | 2.365 | 1.239 |
| 2 | SM(d18:1/26:0 OH) | Sphingomyelin Metabolism | 2.622 | 2.002 | 2.002 | 1.671 |
| 3 | Glycine | 1-Carbon, Folate, Formate, Glycine, Serine Metabolism | 2.523 | 1.891 | 1.891 | 1.392 |
| 4 | 1-Methyladenine | Purine Metabolism | 2.459 | 2.259 | 2.259 | 2.287 |
| 5 | Alanine | Amino Acid Metabolism (not otherwise covered) | 2.456 | 1.687 | 1.687 | 1.322 |
| 6 | Cytosine | Pyrimidine Metabolism | 2.442 | 2.582 | 2.582 | 1.932 |
| 7 | Citric acid | Krebs Cycle | 2.410 | 1.772 | 1.772 | 1.363 |
| 8 | 1-Pyrroline-5-carboxylic acid | GABA, Glutamate, Arginine, Ornithine, Proline Metabolism | 2.358 | 1.922 | 1.922 | 1.299 |
| 9 | Gamma-glutamyl-Alanine | Gamma-Glutamyl and other Dipeptides | 2.353 | 1.725 | 1.725 | 1.644 |
| 10 | Histamine | Histidine, Histamine, Carnosine Metabolism | 2.279 | 1.312 | 1.312 | 1.219 |
| 11 | p-Hydroxyphenylacetic acid | Microbiome Metabolism | 2.203 | 2.226 | 2.226 | 1.306 |
| 12 | Azelaic acid | Nitric Oxide, Superoxide, Peroxide Metabolism | 2.151 | 2.558 | 2.558 | 1.120 |
| 13 | Methionine sulfoxide | SAM, SAH, Methionine, Cysteine, Glutathione Metabolism | 2.104 | 2.083 | 2.083 | 1.409 |
| 14 | L- Kynurenine | Tryptophan, Kynurenine, Serotonin, Melatonin Metabolism | 2.096 | 1.751 | 1.751 | 1.303 |
| 15 | Glycerol 3-phosphate | Glycolysis and Gluconeogenesis Metabolism | 2.081 | 1.731 | 1.731 | 1.294 |
| 16 | Cysteamine | SAM, SAH, Methionine, Cysteine, Glutathione Metabolism | 2.060 | 2.007 | 2.007 | 1.157 |
| 17 | Chenodeoxyglycocholic acid | Bile Salt Metabolism | 2.052 | 1.650 | 1.650 | 2.858 |
| 18 | Hydroxyproline | Vitamin C (Ascorbate) Metabolism | 2.039 | 3.005 | 3.005 | 1.210 |
| 19 | 2-Hydroxyisovaleric acid | Branch Chain Amino Acid Metabolism | 1.988 | 1.146 | 1.146 | 1.234 |
| 20 | Purine | Purine Metabolism | 1.980 | 1.650 | 1.650 | 1.307 |
| 21 | Cyclic adenosine monophosphate | Purine Metabolism | 1.962 | -1.544 | 1.544 | 0.701 |
| 22 | Glycocholic acid | Bile Salt Metabolism | 1.956 | 1.945 | 1.945 | 2.270 |
| 23 | 4-Hydroxyphenyllactic acid | Microbiome Metabolism | 1.945 | 1.172 | 1.172 | 1.294 |
| 24 | Deoxyguanosine diphosphate | Purine Metabolism | 1.915 | -1.583 | 1.583 | 0.864 |
| 25 | Hexose Disaccharide Pool | Amino-Sugar, Galactose, & Non-Glucose Metabolism | 1.911 | 1.220 | 1.220 | 2.121 |
| 26 | S-Adenosylhomocysteine | SAM, SAH, Methionine, Cysteine, Glutathione Metabolism | 1.894 | 0.971 | 0.971 | 1.417 |
| 27 | lsovalerylglycine | Branch Chain Amino Acid Metabolism | 1.888 | 0.901 | 0.901 | 2.027 |
| 28 | Allantoin | Purine Metabolism | 1.882 | 1.068 | 1.068 | 1.798 |
| 29 | Tiglylglycine | Branch Chain Amino Acid Metabolism | 1.878 | 1.310 | 1.310 | 1.302 |
| 30 | L-Phenylalanine | Tyrosine and Phenylalanine Metabolism | 1.875 | 1.381 | 1.381 | 1.245 |
| 31 | cis-aconitic acid | Krebs Cycle | 1.852 | 0.928 | 0.928 | 1.278 |
| 32 | Lathosterol | Cholesterol, Cortisol, Non-Gonadal Steroid Metabolism | 1.844 | 1.079 | 1.079 | 1.284 |
| 33 | L-Asparagine | Amino Acid Metabolism (not otherwise covered) | 1.824 | 1.581 | 1.581 | 1.360 |
| 34 | Cinnamoylglycine | Tyrosine and Phenylalanine Metabolism | 1.790 | 2.218 | 2.218 | 1.190 |
| 35 | Octanoylcarnitine | Fatty Acid Oxidation and Synthesis | 1.780 | -1.451 | 1.451 | 0.703 |
| 36 | L-Cystine | SAM, SAH, Methionine, Cysteine, Glutathione Metabolism | 1.774 | 1.060 | 1.060 | 1.190 |
| 37 | Uridine | Pyrimidine Metabolism | 1.764 | 0.928 | 0.928 | 1.244 |
| 38 | Mevalonic acid | Cholesterol, Cortisol, Non-Gonadal Steroid Metabolism | 1.673 | 1.036 | 1.036 | 1.386 |
| 39 | Chenodeoxycholic acid | Bile Salt Metabolism | 1.670 | 1.575 | 1.575 | 2.080 |
| 40 | Guanidinoacetic acid | SAM, SAH, Methionine, Cysteine, Glutathione Metabolism | 1.644 | 1.254 | 1.254 | 1.217 |
| 41 | 2-Hydroxyisocaproic acid | Branch Chain Amino Acid Metabolism | 1.622 | 0.997 | 0.997 | 1.306 |
| 42 | Decanoylcarnitine | Fatty Acid Oxidation and Synthesis | 1.617 | -1.157 | 1.157 | 0.644 |
| 43 | 3-Hydroxy-cis-5-tetradecenoylcarnitine | Fatty Acid Oxidation and Synthesis | 1.612 | -1.056 | 1.056 | 0.734 |
| 44 | Hippuric acid | Microbiome Metabolism | 1.559 | 0.881 | 0.881 | 1.602 |
| 45 | PE (18:0/18:0) | Phospholipid Metabolism | 1.555 | -1.331 | 1.331 | 0.644 |
| 46 | L-Proline | GABA, Glutamate, Arginine, Ornithine, Proline Metabolism | 1.546 | 0.749 | 0.749 | 1.196 |
| 47 | SM(d18:1/18:2) | Sphingomyelin Metabolism | 1.508 | 0.750 | 0.750 | 1.424 |
| 48 | L-Serine | 1-Carbon, Folate, Formate, Glycine, Serine Metabolism | 1.505 | 0.954 | 0.954 | 1.152 |

## Claims

1. An antipurinergic agent for use in a method of treating an autism spectrum disorder in a subject in need thereof, wherein the method comprises:
(a) administering the antipurinergic agent to said subject in an amount sufficient to maintain the antipurinergic agent plasma levels of said subject within a range of from about 3 µM to about 30 µM for 21 days or more; or
(b) administering the antipurinergic agent to said subject in an amount sufficient to maintain the antipurinergic agent plasma levels of said subject from about 1 µM to about 30 µM for at least about 21 days, wherein said antipurinergic agent is administered in two or more doses; or
(c) administering the antipurinergic agent to said subject in a first dose followed by a 10-40 day treatment holiday and then administering a second dose of said antipurinergic agent.

2. The antipurinergic agent for use of claim 1, wherein (a) applies, and wherein:
the antipurinergic agent plasma levels of said subject are maintained within a range of from about 3 µM to about 15 µM for 21 days or more; or
the antipurinergic agent plasma levels of said subject are maintained within a range of from about 3 µM to about 15 µM for 28 days or more; or
the antipurinergic agent plasma levels of said subject are maintained within a range of from about 3 µM to about 15 µM for 45 days or more; or
the antipurinergic agent plasma levels of said subject are maintained within a range of from about 3 µM to about 15 µM for 60 days or more; or
the antipurinergic agent plasma levels of said subject are maintained within a range of from about 3 µM to about 15 µM for 90 days or more; or
the antipurinergic agent plasma levels of said subject are maintained within a range of from about 5 µM to about 15 µM for 21 days or more; or
the antipurinergic agent plasma levels of said subject are maintained within a range of from about 5 µM to about 15 µM for 60 days or more; or
the antipurinergic agent plasma levels of said subject are maintained within a range of from about 5 µM to about 15 µM for 90 days or more.

3. The antipurinergic agent for use of claim 1, wherein (a) applies, or of claim 2, wherein said administration occurs two or more times.

4. The antipurinergic agent for use of claim 1, wherein (a) applies, or of claim 2 or 3, wherein said antipurinergic agent is administered intravenously.

5. The antipurinergic agent for use of claim 1, wherein (b) applies, and wherein:
said antipurinergic agent plasma levels of said subject are maintained within a range of from about 1 µM to about 15 µM for 21 days or more; or
said antipurinergic agent plasma levels of said subject are maintained within a range of from about 3 µM to about 15 µM for 21 days or more; or
said antipurinergic agent plasma levels of said subject are maintained within a range of from about 5 µM to about 15 µM for 21 days or more.

6. The antipurinergic agent for use of claim 1, wherein (b) applies, or of claim 5, wherein said antipurinergic agent is administered in three or more doses.

7. The antipurinergic agent for use of claim 1, wherein (b) applies, or of claim 5 or 6, wherein said doses of antipurinergic agent are administered intravenously.

8. The antipurinergic agent for use of claim 1, wherein (c) applies, and wherein:
said first dose is within a range from about 10 mg/kg to about 30 mg/kg; or
said first dose is about 5-20 mg/kg; or
said first dose is within a range from about 400 mg/m² to about 600 mg/m².

9. The antipurinergic agent for use of claim 1, wherein (c) applies, or of claim 8, wherein:
said second dose is within a range from about 10 mg/kg to about 30 mg/kg; or
said second dose is about 5-20 mg/kg; or
said second dose is within a range from about 400 mg/m² to about 600 mg/m².

10. The antipurinergic agent for use of claim 1, wherein (c) applies, or of claim 8 or 9, wherein said treatment holiday is within a range from about 12 to about 20 days.

11. The antipurinergic agent for use of claim 1, wherein (c) applies, or of any one of claims 8-10, wherein the method further comprises a 10-60 day treatment holiday after the second dose, followed by a third dose.

12. The antipurinergic agent for use of claim 1, wherein the second dose and the third dose are the same.

13. The antipurinergic agent for use of claim 1, wherein the third dose is smaller than the second dose.

## Patentansprüche

1. Antipurinerges Mittel für die Verwendung für ein Verfahren zum Behandeln einer Autismus-Spektrum-Störung bei einem Subjekt, das dessen bedarf, wobei das Verfahren umfasst:
(a) Verabreichen des antipurinergen Mittels an das Subjekt in einer Menge, die ausreicht, um Plasmaspiegel des antipurinergen Mittels des Subjekts in einem Bereich von etwa 3 µM bis etwa 30 µM für 21 Tage oder länger zu halten; oder
(b) Verabreichen des antipurinergen Mittels an das Subjekt in einer Menge, die ausreicht, um Plasmaspiegel des antipurinergen Mittels des Subjekts von etwa 1 µM bis etwa 30 µM für wenigstens etwa 21 Tage zu halten, wobei das antipurinerge Mittel in zwei oder mehr Dosen verabreicht wird; oder
(c) Verabreichen des antipurinergen Mittels an das Subjekt in einer ersten Dosis, gefolgt von einer 10-40 Tage Behandlungspause und dann Verabreichen einer zweiten Dosis des antipurinergen Mittels.

2. Antipurinerges Mittel für die Verwendung nach Anspruch 1, wobei (a) zutrifft und wobei:
die Plasmaspiegel des antipurinergen Mittels des Subjekts in einem Bereich von etwa 3 µM bis etwa 15 µM für 21 Tage oder länger gehalten werden; oder
die Plasmaspiegel des antipurinergen Mittels des Subjekts in einem Bereich von etwa 3 µM bis etwa 15 µM für 28 Tage oder länger gehalten werden; oder
die Plasmaspiegel des antipurinergen Mittels des Subjekts in einem Bereich von etwa 3 µM bis etwa 15 µM für 45 Tage oder länger gehalten werden; oder
die Plasmaspiegel des antipurinergen Mittels des Subjekts in einem Bereich von etwa 3 µM bis etwa 15 µM für 60 Tage oder länger gehalten werden; oder
die Plasmaspiegel des antipurinergen Mittels des Subjekts in einem Bereich von etwa 3 µM bis etwa 15 µM für 90 Tage oder länger gehalten werden; oder
die Plasmaspiegel des antipurinergen Mittels des Subjekts in einem Bereich von etwa 5 µM bis etwa 15 µM für 21 Tage oder länger gehalten werden; oder
die Plasmaspiegel des antipurinergen Mittels des Subjekts in einem Bereich von etwa 5 µM bis etwa 15 µM für 60 Tage oder länger gehalten werden; oder
die Plasmaspiegel des antipurinergen Mittels des Subjekts in einem Bereich von etwa 5 µM bis etwa 15 µM für 90 Tage oder länger gehalten werden.

3. Antipurinerges Mittel für die Verwendung nach Anspruch 1, wobei (a) zutrifft, oder nach Anspruch 2, wobei die Verabreichung zwei oder mehr Mal erfolgt.

4. Antipurinerges Mittel für die Verwendung nach Anspruch 1, wobei (a) zutrifft, oder nach Anspruch 2 oder 3, wobei das antipurinerge Mittel intravenös verabreicht wird.

5. Antipurinerges Mittel für die Verwendung nach Anspruch 1, wobei (b) zutrifft und wobei:
die Plasmaspiegel des antipurinergen Mittels des Subjekts in einem Bereich von etwa 1 µM bis etwa 15 µM für 21 Tage oder länger gehalten werden; oder
die Plasmaspiegel des antipurinergen Mittels des Subjekts in einem Bereich von etwa 3 µM bis etwa 15 µM für 21 Tage oder länger gehalten werden; oder
die Plasmaspiegel des antipurinergen Mittels des Subjekts in einem Bereich von etwa 5 µM bis etwa 15 µM für 21 Tage oder länger gehalten werden.

6. Antipurinerges Mittel für die Verwendung nach Anspruch 1, wobei (b) zutrifft, oder nach Anspruch 5, wobei das antipurinerge Mittel in drei oder mehr Dosen verabreicht wird.

7. Antipurinerges Mittel für die Verwendung nach Anspruch 1, wobei (b) zutrifft, oder nach Anspruch 5 oder 6, wobei die Dosen des antipurinergen Mittels intravenös verabreicht werden.

8. Antipurinerges Mittel für die Verwendung nach Anspruch 1, wobei (c) zutrifft und wobei:
die erste Dosis in einem Bereich von etwa 10 mg/kg bis etwa 30 mg/kg liegt; oder
die erste Dosis etwa 5-20 mg/kg beträgt; oder
die erste Dosis in einem Bereich von etwa 400 mg/m² bis etwa 600 mg/m² liegt.

9. Antipurinerges Mittel für die Verwendung nach Anspruch 1, wobei (c) zutrifft, oder nach Anspruch 8, wobei:
die zweite Dosis in einem Bereich von etwa 10 mg/kg bis etwa 30 mg/kg liegt; oder
die zweite Dosis etwa 5-20 mg/kg beträgt; oder
die zweite Dosis in einem Bereich von etwa 400 mg/m² bis etwa 600 mg/m² liegt.

10. Antipurinerges Mittel für die Verwendung nach Anspruch 1, wobei (c) zutrifft, oder nach Anspruch 8 oder 9, wobei die Behandlungspause in einem Bereich von etwa 12 bis etwa 20 Tagen liegt.

11. Antipurinerges Mittel für die Verwendung nach Anspruch 1, wobei (c) zutrifft oder nach einem der Ansprüche 8-10, wobei das Verfahren ferner eine 10-60 Tage Behandlungspause nach der zweiten Dosis umfasst, gefolgt von einer dritten Dosis.

12. Antipurinerges Mittel für die Verwendung nach Anspruch 1, wobei die zweite Dosis und die dritte Dosis dieselbe sind.

13. Antipurinerges Mittel für die Verwendung nach Anspruch 1, wobei die dritte Dosis kleiner als die zweite Dosis ist.

## Revendications

1. Agent antipurinergique pour l'utilisation dans un procédé de traitement de trouble du spectre de l'autisme chez un sujet en ayant besoin, dans lequel le procédé comprend :
(a) l'administration de l'agent antipurinergique audit sujet en une quantité suffisante pour maintenir les niveaux plasmatiques d'agent antipurinergique dudit sujet au sein d'une plage d'environ 3 µM à environ 30 µM pendant 21 jours ou plus ; ou
(b) l'administration de l'agent antipurinergique audit sujet en une quantité suffisante pour maintenir les niveaux plasmatiques d'agent antipurinergique dudit sujet d'environ 1 µM à environ 30 µM pendant au moins environ 21 jours, dans lequel ledit agent antipurinergique est administré en deux doses, ou plus ; ou
(c) l'administration de l'agent antipurinergique audit sujet en une première dose suivie par une fenêtre thérapeutique de 10 à 40 jours, et puis l'administration d'une deuxième dose dudit agent antipurinergique.

2. Agent antipurinergique pour l'utilisation selon la revendication 1, dans lequel (a) est appliquée, et dans lequel :
les niveaux plasmatiques d'agent antipurinergique dudit sujet sont maintenus au sein d'une plage d'environ 3 µM à environ 15 µM pendant 21 jours ou plus ; ou
les niveaux plasmatiques d'agent antipurinergique dudit sujet sont maintenus au sein d'une plage d'environ 3 µM à environ 15 µM pendant 28 jours ou plus ; ou
les niveaux plasmatiques d'agent antipurinergique dudit sujet sont maintenus au sein d'une plage d'environ 3 µM à environ 15 µM pendant 45 jours ou plus ; ou
les niveaux plasmatiques d'agent antipurinergique dudit sujet sont maintenus au sein d'une plage d'environ 3 µM à environ 15 µM pendant 60 jours ou plus ; ou
les niveaux plasmatiques d'agent antipurinergique dudit sujet sont maintenus au sein d'une plage d'environ 3 µM à environ 15 µM pendant 90 jours ou plus ; ou
les niveaux plasmatiques d'agent antipurinergique dudit sujet sont maintenus au sein d'une plage d'environ 5 µM à environ 15 µM pendant 21 jours ou plus ; ou
les niveaux plasmatiques d'agent antipurinergique dudit sujet sont maintenus au sein d'une plage d'environ 5 µM à environ 15 µM pendant 60 jours ou plus ; ou
les niveaux plasmatiques d'agent antipurinergique dudit sujet sont maintenus au sein d'une plage d'environ 5 µM à environ 15 µM pendant 90 jours ou plus.

3. Agent antipurinergique pour l'utilisation selon la revendication 1, dans lequel (a) est appliquée, ou selon la revendication 2, dans lequel ladite administration a lieu deux fois ou plus.

4. Agent antipurinergique pour l'utilisation selon la revendication 1, dans lequel (a) est appliquée, ou selon la revendication 2 ou 3, dans lequel ledit agent antipurinergique est administré de façon intraveineuse.

5. Agent antipurinergique pour l'utilisation selon la revendication 1, dans lequel (b) est appliquée, et dans lequel :
lesdits niveaux plasmatiques d'agent antipurinergique dudit sujet sont maintenus au sein d'une plage d'environ 1 µM à environ 15 µM pendant 21 jours ou plus ; ou
lesdits niveaux plasmatiques d'agent antipurinergique dudit sujet sont maintenus au sein d'une plage d'environ 3 µM à environ 15 µM pendant 21 jours ou plus ; ou
lesdits niveaux plasmatiques d'agent antipurinergique dudit sujet sont maintenus au sein d'une plage d'environ 5 µM à environ 15 µM pendant 21 jours ou plus.

6. Agent antipurinergique pour l'utilisation selon la revendication 1, dans lequel (b) est appliquée, ou selon la revendication 5, dans lequel ledit agent antipurinergique est administré en trois doses, ou plus.

7. Agent antipurinergique pour l'utilisation selon la revendication 1, dans lequel (b) est appliquée, ou selon la revendication 5 ou 6, dans lequel lesdites doses d'agent antipurinergique sont administrées de façon intraveineuse.

8. Agent antipurinergique pour l'utilisation selon la revendication 1, dans lequel (c) est appliquée, et dans lequel :
ladite première dose est au sein d'une plage d'environ 10 mg/kg à environ 30 mg/kg ; ou
ladite première dose est environ 5 à 20 mg/kg ; ou
ladite première dose est au sein d'une plage d'environ 400 mg/m² à environ 600 mg/m².

9. Agent antipurinergique pour l'utilisation selon la revendication 1, dans lequel (c) est appliquée, ou selon la revendication 8, dans lequel :
ladite deuxième dose est au sein d'une plage d'environ 10 mg/kg à environ 30 mg/kg ; ou
ladite deuxième dose est environ 5 à 20 mg/kg ; ou
ladite deuxième dose est au sein d'une plage d'environ 400 mg/m² à environ 600 mg/m².

10. Agent antipurinergique pour l'utilisation selon la revendication 1, dans lequel (c) est appliquée, ou selon la revendication 8 ou 9, dans lequel ladite fenêtre thérapeutique est au sein d'une plage d'environ 12 à environ 20 jours.

11. Agent antipurinergique pour l'utilisation selon la revendication 1, dans lequel (c) est appliquée, ou selon l'une quelconque des revendications 8 à 10, dans lequel le procédé comprend en outre une fenêtre thérapeutique de 10 à 60 jours après la deuxième dose, suivie par une troisième dose.

12. Agent antipurinergique pour l'utilisation selon la revendication 1, dans lequel la deuxième dose et la troisième dose sont les mêmes.

13. Agent antipurinergique pour l'utilisation selon la revendication 1, dans lequel la troisième dose est plus petite que la deuxième dose.
